# EUROPEAN PATENT APPLICATION

(11) **EP 4 843 483 A2**
(43) Date of publication of application: **25.11.2026**
(21) Application number: 26210242.9
(22) Date of filing: 21.12.2023
(51) Int. Cl.: A61B 5/00

(54) **SYSTEMS, DEVICES, AND METHODS FOR DUAL ANALYTE SENSOR**

(30) Priority: 23.12.2022 US 202263435028 P
(62) Document number(s) of the earlier application(s) in accordance with Art. 76 EPC: 23848502.3 / 4 639 572
(71) Applicant: Abbott Diabetes Care Inc., Alameda, California 94502 (US)
(72) Inventor: HAYTER, Gary A., Oakland, 94618 (US); BUDIMAN, Erwin S., Fremont, 94555 (US); TAUB, Marc B., Mountain View, 94040 (US); HARPER, Wesley Scott, Alameda, 94502 (US)
(74) Representative: J A Kemp LLP

(57) **Abstract**

An analyte monitoring system and method for an on-body sensor control device may have a capability for sensing two or more analytes of a patient includes a processor and memory with instructions for determining various machine states and outputs based on time-correlated sensor data for each of the analytes. The states and outputs may include: an alert condition for one or more analytes; an advisory message; a correction to an analyte state estimate; an indication of a malfunction of the sensor control device, a malfunction of an insulin delivery device, a medication dose calculation; or an analytical report. Examples of the system and method for dual analytes comprising glucose and ketone are described. Data may be transmitted from the sensor control device to a reader device for output to a user.

## Description

### CROSS-REFERENCE TO RELATED APPLICATION

The present application claims priority to U.S. Provisional Application Serial No. 63/435,028 filed December 23, 2022, which is hereby expressly incorporated by reference in its entirety for all purposes.

### FIELD

The subject matter described herein relates generally to systems, devices, and methods for a dual analyte sensor. In particular, the embodiments described herein involve using data collected by a glucose sensor with data collected from a ketone sensor, for controlling a user interface device or dose administration device for improved control of a patient's glucose level.

### BACKGROUND

A large and growing market exists for monitoring the health and condition of humans and other living animals. Information that describes the physical or physiological condition of humans can be used in countless ways to assist and improve quality of life and diagnose and treat undesirable human conditions.

A common device used to collect such information is a physiological sensor such as a biochemical analyte sensor, or a device capable of sensing a chemical analyte of a biological entity. Biochemical sensors come in many forms and can be used to sense analytes in fluids, tissues, or gases forming part of, or produced by, a biological entity, such as a human being. These analyte sensors can be used on or within the body itself, such as in the case of a transcutaneously implanted analyte sensor, or they can be used on biological substances that have already been removed from the body. Useful applications for such sensors include blood glucose sensing for purpose of health assessment, dose guidance, and related uses.

However, blood glucose monitoring alone is subject to certain limitations. For example, patients with T1 diabetes using SGLT-2i inhibitors, also called gliflozins or flozins, are at risk of experiencing euglycemic diabetic ketoacidosis (DKA). DKA is an adverse condition concerning to diabetes patients, which can result in hospitalization or even death. It is associated with high ketone levels that are caused by insufficient glucose uptake in insulin-dependent cells, evident from long durations of high glucose levels. Glucose uptake insufficiency resulting in DKA can be caused by insufficient insulin levels in the patient or high levels of insulin resistance, perhaps caused by illness - in this case, the glucose levels may be in the target range or below.

SGLT-2i is a diabetes medication that helps reduce glucose variability around meal-times and is indicated for use on patients with T2 diabetes. It also can help patients with T1 diabetes in managing their glucose levels; however, there is a concern in using it for T1 patients because of the possibility of causing high ketone levels and DKA, with normal levels of glucose, referred to herein as euglycemic DKA. For people with T1 diabetes, sotagliflozin, an SGLT-1 and SGLT-2i inhibitor, is the only medication containing SGLT-2i approved by the European Medicines Agency. Therefore, continuous ketone monitoring can be an important component in managing T2 diabetes with SGLT-2i, and potentially mitigate the euglycemic DKA risk in people with T1 diabetes taking SGLT-2i medication.

Treatment for euglycemic DKA is basically to administer insulin and to offset any unwanted glucose lowering impact by consuming carbohydrates. However, it can be confusing to patients when they should do this and when they should seek emergency medical intervention. Specifically, it can be confusing to know what to do when their ketones are elevated but not high enough to represent DKA, and their glucose levels are in the normal range or low. In addition, the patient's HCP would benefit from contextual information before and after an elevated or high ketone episode, in order to better understand the cause of this condition and, if needed, help mitigate it.

Discrete ketone test strips, along with continuous glucose monitoring (CGM), are available but may not be practical for timely monitoring of ketones. A particular high glucose reading is not always associated with a particular high ketone reading, which may require some guesswork in determining when to take ketone strip reading. Regardless of how the patient's ketone is measured, interpretation of the ketone level in conjunction with the patient's blood glucose levels and determination of appropriate action is too complex for most patients, requiring input from a health care provider (HCP). Accordingly, ketone sensing and use of ketone data by patients taking SGLT-2i inhibitors is relatively difficult and cumbersome, compared to continuous glucose sensing.

For these and other reasons, needs exist for improving ketone sensing, analysis, and guidance for patients vulnerable to euglycemic DKA, for example, patients taking SGLT-2i inhibitors.

### SUMMARY

Example embodiments of systems, devices, and methods are described herein for a dual analyte sensor using glucose history from a glucose sensor in combination with data from a ketone sensor for controlling a user interface device, health care provider interface device, or dose administration device for improved control of a patient's glucose level.

An analyte monitoring system and method for an on-body sensor control device may have a capability for sensing two or more analytes of a patient includes a processor and memory with instructions for determining various machine states and outputs based on time-correlated sensor data for each of the analytes. The states and outputs may include: an alert condition for one or more analytes; an advisory message; a correction to an analyte state estimate; an indication of a malfunction of the sensor control device, a malfunction of an insulin delivery device, a medication dose calculation; or an analytical report. Examples of the system and method for dual analytes comprising glucose and ketone are described. Data may be transmitted from the sensor control device to a reader device for output to a user.

The present disclosure describes mobile application-based systems, apparatus, and methods for detecting conditions where actions should be taken, providing guidance to the patient and providing a means to record and output important context concurrent with the condition that will be helpful for the HCP to know later when advising the patient how to avoid the adverse condition in the future. In an aspect, the systems, apparatus or method may include features for generating an estimate of the patient's medication state and/or knowledge of medication information (e.g., a patient with T1 diabetes mellitus (DM) using an SGLT-2i inhibitor. In an alternative, or in addition, the systems, apparatus or method may deliver guidance to the patient at more appropriate times, and capture additional information about the patient's condition and contexts at moments when the patient can remember and provide the information.

In addition, or in an alternative, an improved system, method or apparatus may include improving an alert feature of an analyte monitoring sensor (e.g., glucose sensor) by using context from a ketone sensor and/or estimate of medication state and/or knowledge of medication information (e.g., a person with T1 DM using an SGLT-2i inhibitor). In comparison, single analyte sensor systems may include, for example, a high threshold alert, and a low projected threshold alert. The single-analyte alert may be improved by adjusting the alert behavior and timing using data from a ketone sensor. This may include dual glucose-ketone analyte systems for which threshold alerts are based on each analyte's value, independent of other analyte values and/or medication based information. Examples of adjusting alert behavior include using a lower or higher threshold. An example of adjusting timing include changing the alert enunciation time interval when the alert condition is still met. Adjusting of timing improves the clinical relevance of the alert and may reduce alert fatigue by minimizing enunciation that may be less clinically relevant.

The systems, apparatus and methods disclosed herein may incorporate ketone data together with blood glucose data to provide patient and HCP guidance that is more reliable than using high glucose threshold detection alone. On-demand systems or continuous glucose monitoring (CGM) systems may thus be provided with improved utility. For example, an on-demand test system including a built-in ketone-measurement-compatible strip port can provide an enhanced utility to the patient and assist the HCP in making more accurate recommendations. In general, the systems, apparatus and methods disclosed herein may better protect the patient from DKA risk. Algorithmic improvements in the systems, methods and apparatus may include utilization of rich glucose history from on-demand or CGM systems, opportunistic use of insulin history (e.g., from a built-in bolus calculator) and ongoing ketone measurement (e.g., from built-in ketone compatible strip port or in vivo ketone analyte sensor) to improve future DKA risk estimation, and improved DKA risk assessment. Improved risk assessment algorithms may include, for example, comparing estimated ketone time series to a ketone-specific threshold, instead of comparing point glucose levels to a conservative point glucose specific threshold as is conventionally done.

According to some embodiments, an analyte monitoring system comprises: a sensor control device including an analyte sensor, first processing circuitry, and a first non-transitory memory, wherein the analyte sensor includes at least a portion configured to be inserted into a user's body, and wherein the sensor control device is configured to collect first time-correlated data indicative of a glucose level and second time-correlated data indicative of a ketone level; and a reader device comprising second processing circuitry and a second non-transitory memory, wherein at least one of the first or the second non-transitory memory includes instructions that, when executed, cause at least one of the first or the second processing circuitry to perform: determining, based at least in part on the first and second time-correlated data, at least one of: an alert condition for one or both of the first and second time-correlated data, an advisory message for output by the reader device, a correction to an analyte state estimate, a malfunction of the sensor control device, a malfunction of an insulin delivery device, a medication dose calculation, or an analytical report based on the first and second time-correlated data; and outputting, by the reader device, an indication of an outcome of the determining.

According to some embodiments, a computer-implemented method for analyte monitoring and control for a patient comprises: collecting, by a sensor control device, first time-correlated data indicative of a glucose level and second time-correlated data indicative of a ketone level, wherein the sensor control device includes an analyte sensor at least a portion of which is inserted into a user's body; determining, based at least in part on the first and second time-correlated data, at least one of: an alert condition for one or both of the first and second time-correlated data, an advisory message for output by the reader device, a correction to an analyte state estimate, a malfunction of the sensor control device, a malfunction of an insulin delivery device, a medication dose calculation, or an analytical report based on the first and second time-correlated data; and outputting, by a user interface device, an indication of an outcome of the determining.

Other systems, devices, methods, features and advantages of the subject matter described herein will be or will become apparent to one with skill in the art upon examination of the following figures and detailed description. It is intended that all such additional systems, methods, features and advantages be included within this description, be within the scope of the subject matter described herein, and be protected by the accompanying claims. **In** no way should the features of the example embodiments be construed as limiting the appended claims, absent express recitation of those features in the claims.

### BRIEF DESCRIPTION OF FIGURES

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
FIG. 1 is an illustrative view depicting an example embodiment of an in vivo analyte monitoring system.
FIG. 2 is a block diagram of an example embodiment of a reader device.
FIG. 3 is a block diagram of an example embodiment of a sensor control device.
FIG. 4 is a table illustrating conditional logic for formulating an advisory message.
FIG. 5 is a flow diagram depicting an example embodiment of a method for analyte monitoring and control.

### DETAILED DESCRIPTION

Before the present subject matter is described in detail, it is to be understood that this disclosure is not limited to the particular embodiments described, as such may vary. It is also to be understood that the terminology used herein is for the purpose of describing particular embodiments only and is not intended to be limiting. The scope of the present disclosure will be limited only by the appended claims.

The publications discussed herein are provided solely for their disclosure prior to the filing date of the present application. Nothing herein is to be construed as an admission that the present disclosure is not entitled to antedate such publications by virtue of prior disclosure. Furthermore, the dates of publication provided may be different from the actual publication dates which may need to be independently confirmed.

Generally, embodiments of the present disclosure are used with systems, devices, and methods for detecting at least one analyte, such as glucose, in a bodily fluid (e.g., subcutaneously within the interstitial fluid ("ISF") or blood, within the dermal fluid of the dermal layer, or otherwise), in conjunction with a feature for ketone analyte sensing that is chronologically correlated to the analyte data from an in vivo glucose sensor. Embodiments may include in vivo analyte sensors structurally configured so that at least a portion of the sensors are, or can be, positioned in the body of a user to obtain information about glucose and ketone analytes of the body. However, the embodiments disclosed herein can be used with in vivo analyte monitoring systems that incorporate in vitro capability, as well as purely in vitro or ex vivo analyte monitoring systems, including those systems that are entirely non-invasive. If used with a single-analyte in vivo sensor, ketone test data may be added manually, for example by using a test strip. **In** an alternative, embodiments of the present disclosure may be used with dual-sensor systems for continuous or semi-continuous monitoring of different analytes, for example blood glucose and ketone bodies.

Furthermore, for each embodiment of a method disclosed herein, systems and devices capable of performing each of those embodiments are covered within the scope of the present disclosure. For example, embodiments of sensor control devices are disclosed and these devices can have one or more sensors, analyte monitoring circuitry (e.g., an analog circuit), non-transitory memories (e.g., for storing instructions), power sources, communication circuitry, transmitters, receivers, processing circuitry, and/or controllers (e.g., for executing instructions) that can perform any and all method steps or facilitate the execution of any and all method steps. These sensor control device embodiments can be used and can be capable of use to implement those steps performed by a sensor control device from one or more of the methods described herein.

Likewise, embodiments of reader devices are disclosed having one or more transmitters, receivers, non-transitory memories (e.g., for storing instructions), power sources, processing circuitry, and/or controllers (e.g., for executing instructions) that can perform any and all method steps or facilitate the execution of any and all method steps. These embodiments of the reader devices can be used to implement those steps performed by a reader device from one or more of the methods described herein.

Embodiments of trusted computer systems are also disclosed. These trusted computer systems can include one or more processing circuits, controllers, transmitters, receivers, non-transitory memories, databases, servers, and/or networks, and can be discretely located or distributed across multiple geographic locales. These embodiments of the trusted computer systems can be used to implement those steps performed by a trusted computer system from one or more of the methods described herein.

Various embodiments of systems, devices and methods for analyte monitoring and control are disclosed. According to some embodiments, these systems, devices, and methods can utilize a first data collected by a glucose sensor and a second data collected by a ketone sensing element.

Other features and advantages of the disclosed embodiments are further discussed below.

Before describing the embodiments in detail, however, it is first desirable to describe examples of devices that can be present within, for example, an in vivo analyte monitoring system, as well as examples of their operation, all of which can be used with the embodiments described herein.

### Example Embodiments of Analyte Monitoring Systems

There are various types of analyte monitoring systems. "Continuous Analyte Monitoring" systems (or "Continuous Glucose Monitoring" systems), for example, are in vivo systems that can transmit data from a sensor control device to a reader device repeatedly or continuously without prompting, e.g., automatically according to a schedule. "Flash Analyte Monitoring" systems (or "Flash Glucose Monitoring" systems or simply "Flash" systems), as another example, are in vivo systems that can transfer data from a sensor control device in response to a scan or request for data by a reader device, such as with a Near Field Communication (NFC) or Radio Frequency Identification (RFID) protocol. In vivo analyte monitoring systems can also operate without the need for finger stick calibration.

In vivo monitoring systems can include a sensor that, while positioned in vivo, contacts the bodily fluid of the user and senses one or more analyte levels contained therein. The sensor can be part of a sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit (OBU), or a "sensor data communication" device or unit, for example. As used herein, these terms are not limited to devices with analyte sensors, and encompass devices that have sensors of other types, whether biometric or non-biometric. The term "on body" refers to any device that resides directly on the body or in close proximity to the body, such as a wearable device (e.g., glasses, watch, wristband or bracelet, neckband or necklace, etc.).

In vivo monitoring systems can also include one or more reader devices that receive sensed analyte data from the sensor control device. These reader devices can process and/or display the sensed analyte data, or sensor data, in any number of forms, to the user. These devices, and variations thereof, can be referred to as "user interface devices," "handheld reader devices," "reader devices" (or simply, "readers"), "handheld electronics" (or handhelds), "portable data processing" devices or units, "data receivers," "receiver" devices or units (or simply receivers), "relay" devices or units, or "remote" devices or units, to name a few. Other devices such as personal computers have also been utilized with or incorporated into in vivo and in vitro monitoring systems.

In vivo analyte monitoring systems can be differentiated from "in vitro" systems that contact a biological sample outside of the body (or rather "ex vivo") and that typically include a meter device that has a port for receiving an analyte test strip carrying a bodily fluid of the user, which can be analyzed to determine the user's analyte level. As mentioned, the embodiments described herein can be used with in vivo systems, in vitro systems, and combinations thereof.

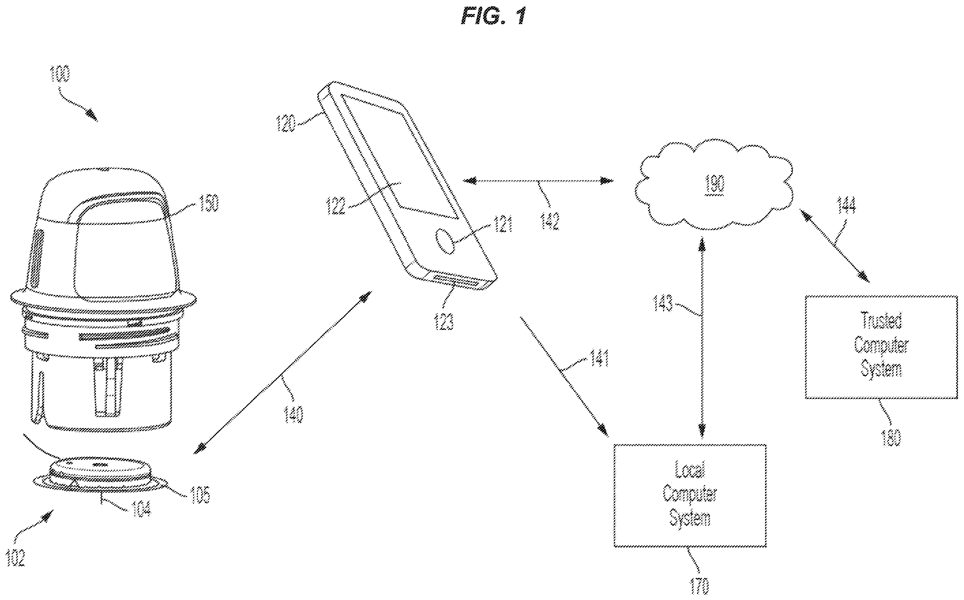

FIG. 1 is an illustrative view depicting an example embodiment of an in vivo analyte monitoring system 100 having a sensor control device 102 and a reader device 120 that communicate with each other over a local communication path (or link) 140, which can be wired or wireless, and uni-directional or bi-directional. In embodiments where the path 140 is wireless, a near field communication (NFC) protocol, RFID protocol, Bluetooth or Bluetooth Low Energy protocol, Wi-Fi protocol, proprietary protocol, or the like can be used, including those communication protocols in existence as of the date of this filing or their later developed variants.

Reader device 120 is also capable of wired, wireless, or combined communication with a computer system 170 (e.g., a local or remote computer system) over communication path (or link) 141 and with a network 190, such as the internet or the cloud, over communication path (or link) 142. Communication with network 190 can involve communication with trusted computer system 180 within network 190, or though network 190 to computer system 170 via communication link (or path) 143. Communication paths 141, 142, and 143 can be wireless, wired, or both, can be uni-directional or bi-directional, and can be part of a telecommunications network, such as a Wi-Fi network, a local area network (LAN), a wide area network (WAN), the internet, or other data network. In some cases, communication paths 141 and 142 can be the same path. All communications over paths 140, 141, and 142 can be encrypted and sensor control device 102, reader device 120, computer system 170, and trusted computer system 180 can each be configured to encrypt and decrypt those communications sent and received.

Variants of devices 102 and 120, as well as other components of an in vivo-based analyte monitoring system that are suitable for use with the system, device, and method embodiments set forth herein, are described in U.S. Patent Publication No. 2011/0213225 (the '225 Publication), which is incorporated by reference herein in its entirety for all purposes.

Sensor control device 102 can include a housing 103 containing in vivo analyte monitoring circuitry and a power source. **In** this embodiment, the in vivo analyte monitoring circuitry is electrically coupled with one or more analyte sensors 104, 106 that extend through an adhesive patch 105 and projects away from housing 103. The sensors may include a blood glucose sensor 104 and a ketone sensor 106. An adhesive patch 105 may include an adhesive layer (not shown) for attachment to a skin surface of the body of the user. Other forms of body attachment to the body may be used, in addition to or instead of adhesive.

A glucose sensor 104 and optionally, a ketone sensor 106, may be adapted to be at least partially inserted into the body of the user, where it can make fluid contact with that user's bodily fluid (e.g., subcutaneous (subdermal) fluid, dermal fluid, or blood) and be used, along with the in vivo analyte monitoring circuitry, to measure analyte-related data of the user. Sensors 104, 106 and any accompanying sensor control electronics can be applied to the body in any desired manner. For example, an insertion device 150 can be used to position all or a portion of analyte sensor 104 through an external surface of the user's skin and into contact with the user's bodily fluid. **In** doing so, the insertion device can also position sensor control device 102 with adhesive patch 105 onto the skin. **In** other embodiments, insertion device can position sensor 104 first, and then accompanying sensor control electronics can be coupled with sensor 104 afterwards, either manually or with the aid of a mechanical device. Examples of insertion devices are described in U.S. Publication Nos. 2008/0009692, 2011/0319729, 2015/0018639, 2015/0025345, and 2015/0173661, all which are incorporated by reference herein in their entireties and for all purposes.

After collecting raw data from the user's body, sensor control device 102 can apply analog signal conditioning to the data and convert the data into a digital form of the conditioned raw data. **In** some embodiments, sensor control device 102 can then algorithmically process the digital raw data into a form that is representative of the user's measured biometric (e.g., analyte level) and/or one or more analyte metrics based thereupon. For example, sensor control device 102 can include processing circuitry to algorithmically perform any of the method steps described herein. Sensor control device 102 can then encode and wirelessly communicate data indicative of a glucose level, a ketone level, indications of sensor fault and/or processed sensor data to reader device 120, which in turn can format or graphically process the received data for digital display to the user. **In** other embodiments, in addition to, or in lieu of, wirelessly communicating sensor data to another device (e.g., reader device 120), sensor control device 102 can graphically process the final form of the data such that it is ready for display, and display that data on a display of sensor control device 102. **In** some embodiments, the final form of the biometric data (prior to graphic processing) is used by the system (e.g., incorporated into a diabetes monitoring regime) without processing for display to the user.

**In** still other embodiments, the conditioned raw digital data can be encoded for transmission to another device, e.g., reader device 120, which then algorithmically processes that digital raw data into a form representative of the user's measured biometric (e.g., a form readily made suitable for display to the user) and/or one or more analyte metrics based thereupon. Reader device 120 can include processing circuitry to algorithmically perform any of the method steps described herein such as, for example, to correct a glucose level measurement, to detect a suspected glucose dropout, or to detect a suspected sensor fault condition, or other operation. This algorithmically processed data can then be formatted or graphically processed for digital display to the user.

**In** other embodiments, sensor control device 102 and reader device 120 transmit the digital raw data to another computer system for algorithmic processing and display.

Reader device 120 can include a display 122 to output information to the user and/or to accept an input from the user, and an optional input component 121 (or more), such as a button, actuator, touch sensitive switch, capacitive switch, pressure sensitive switch, jog wheel or the like, to input data, commands, or otherwise control the operation of reader device 120. In certain embodiments, display 122 and input component 121 may be integrated into a single component, for example, where the display can detect the presence and location of a physical contact touch upon the display, such as a touch screen user interface. In certain embodiments, input component 121 of reader device 120 may include a microphone and reader device 120 may include software configured to analyze audio input received from the microphone, such that functions and operation of the reader device 120 may be controlled by voice commands. In certain embodiments, an output component of reader device 120 includes a speaker (not shown) for outputting information as audible signals. Similar voice responsive components such as a speaker, microphone and software routines to generate, process and store voice driven signals may be included in sensor control device 102.

Reader device 120 can also include one or more data communication ports 123 for wired data communication with external devices such as computer system 170 or sensor control device 102. Example data communication ports include USB ports, mini USB ports, USB Type-C ports, USB micro-A and/or micro-B ports, RS-232 ports, Ethernet ports, Firewire ports, or other similar data communication ports configured to connect to the compatible data cables. Reader device 120 may also include an integrated or attachable in vitro glucose meter, including an in vitro test strip port (not shown) to receive an in vitro glucose test strip for performing in vitro blood glucose measurements.

Reader device 120 can display the measured biometric data wirelessly received from sensor control device 102 and can also be configured to output alarms, alert notifications, glucose values, etc., which may be visual, audible, tactile, or any combination thereof. Further details and other display embodiments can be found in, e.g., U.S. Publication No. 2011/0193704, which is incorporated herein by reference in its entirety for all purposes.

Reader device 120 can function as a data conduit to transfer the measured data and/or analyte metrics from sensor control device 102 to computer system 170 or trusted computer system 180. **In** certain embodiments, the data received from sensor control device 102 may be stored (permanently or temporarily) in one or more memories of reader device 120 prior to uploading to system 170, 180 or network 190.

Computer system 170 may be a personal computer, a server terminal, a laptop computer, a tablet, or other suitable data processing device. Computer system 170 can be (or include) software for data management and analysis and communication with the components in analyte monitoring system 100. Computer system 170 can be used by the user or a medical professional to display and/or analyze the biometric data measured by sensor control device 102. **In** some embodiments, sensor control device 102 can communicate the biometric data directly to computer system 170 without an intermediary such as reader device 120, or indirectly using an internet connection (also optionally without first sending to reader device 120). Operation and use of computer system 170 may be as further described in the '225 Publication incorporated herein, with additional method steps for handling ketone data in conjunction with blood glucose data. Analyte monitoring system 100 can also be configured to operate with a data processing module (not shown), also as described in the incorporated '225 Publication.

Trusted computer system 180 can be within the possession of the manufacturer or distributor of sensor control device 102, either physically or virtually through a secured connection, and can be used to perform authentication of sensor control device 102, for secure storage of the user's biometric data, and/or as a server that serves a data analytics program (e.g., accessible via a web browser) for performing analysis on the user's measured data.

### Example Embodiments of Reader Devices

Reader device 120 can be a mobile communication device such as a dedicated reader device (configured for communication with a sensor control device 102, and optionally a computer system 170, but without mobile telephony communication capability) or a mobile telephone including, but not limited to, a Wi-Fi or internet enabled smart phone, tablet, or personal digital assistant (PDA). Examples of smart phones can include those mobile phones based on a Windows^{®} operating system, Android^{™} operating system, iPhone^{®} operating system, Palm^{®} WebOS^{™}, Blackberry^{®} operating system, or Symbian^{®} operating system, with data network connectivity functionality for data communication over an internet connection and/or a local area network (LAN).

Reader device 120 can also be configured as a mobile smart wearable electronics assembly, such as an optical assembly that is worn over or adjacent to the user's eye (e.g., a smart glass or smart glasses, such as Google glasses, which is a mobile communication device). This optical assembly can have a transparent display that displays information about the user's analyte level (as described herein) to the user while at the same time allowing the user to see through the display such that the user's overall vision is minimally obstructed. The optical assembly may be capable of wireless communications similar to a smart phone. Other examples of wearable electronics include devices that are worn around or in the proximity of the user's wrist (e.g., a watch, etc.), neck (e.g., a necklace, etc.), head (e.g., a headband, hat, etc.), chest, or the like.

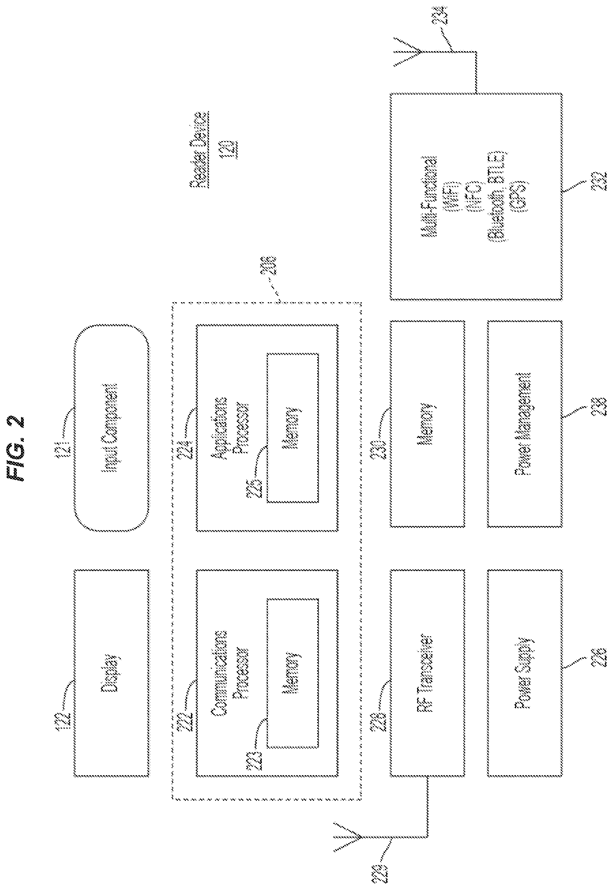

FIG. 2 is a block diagram of an example embodiment of a reader device 120 configured as a smart phone. Here, reader device 120 includes an input component 121, display 122, and processing circuitry 206, which can include one or more processors, microprocessors, controllers, and/or microcontrollers, each of which can be a discrete chip or distributed amongst (and a portion of) a number of different chips. Here, processing circuitry 206 includes a communications processor 222 having on-board memory 223 and an applications processor 224 having on-board memory 225. Reader device 120 further includes RF communication circuitry 228 coupled with an RF antenna 229, a memory 230, multi-functional circuitry 232 with one or more associated antennas 234, a power supply 226, power management circuitry 238, and a clock (not shown). One or more of the memories 223, 225 may hold program instructions, that when executed by one or more of the processors 222, 224, cause the reader device 120 to perform one or more operations of methods described herein. FIG. 2 is an abbreviated representation of the typical hardware and functionality that resides within a smart phone and those of ordinary skill in the art will readily recognize that other hardware and functionality (e.g., codecs, drivers, glue logic) can also be included.

Communications processor 222 can interface with RF communication circuitry 228 and perform analog-to-digital conversions, encoding and decoding, digital signal processing and other functions that facilitate the conversion of voice, video, and data signals into a format (e.g., in-phase and quadrature) suitable for provision to RF communication circuitry 228, which can then transmit the signals wirelessly. Communications processor 222 can also interface with RF communication circuitry 228 to perform the reverse functions necessary to receive a wireless transmission and convert it into digital data, voice, and video. RF communication circuitry 228 can include a transmitter and a receiver (e.g., integrated as a transceiver) and associated encoder logic.

Applications processor 224 can be adapted to execute the operating system and any software applications that reside on reader device 120, process video and graphics, and perform those other functions not related to the processing of communications transmitted and received over RF antenna 229. The smart phone operating system will operate in conjunction with a number of applications on reader device 120. Any number of applications (also known as "user interface applications") can be running on reader device 120 at any one time, and may include one or more applications that are related to a diabetes monitoring regime and methods described herein, in addition to the other commonly used applications that are unrelated to such a regime, e.g., email, calendar, weather, sports, games, etc. For example, the data indicative of a sensed analyte level and in vitro blood analyte measurements received by the reader device can be securely communicated to user interface applications residing in memory 230 of reader device 120. Such communications can be securely performed, for example, by mobile application containerization or wrapping technologies.

Memory 230 can be shared by one or more of the various functional units present within reader device 120, or can be distributed amongst two or more of them (e.g., as separate memories present within different chips). Memory 230 can also be a separate chip of its own. Memories 223, 225, and 230 are non-transitory, and can be volatile (e.g., RAM, etc.) and/or non-volatile memory (e.g., ROM, flash memory, F-RAM, etc.).

Multi-functional circuitry 232 can be implemented as one or more chips and/or components (e.g., transmitter, receiver, transceiver, and/or other communication circuitry) that perform other functions such as local wireless communications, e.g., with sensor control device 102 under the appropriate protocol (e.g., Wi-Fi, Bluetooth, Bluetooth Low Energy, Near Field Communication (NFC), Radio Frequency Identification (RFID), proprietary protocols, and others) and determining the geographic position of reader device 120 (e.g., global positioning system (GPS) hardware). One or more other antennas 234 are associated with the functional circuitry 232 as needed to operate with the various protocols and circuits.

Power supply 226 can include one or more batteries, which can be rechargeable or single-use disposable batteries. Power management circuitry 238 can regulate battery charging and power supply monitoring, boost power, perform DC conversions, and the like.

Reader device 120 can also include or be integrated with a drug (e.g., insulin, etc.) delivery device such that they, e.g., share a common housing. Examples of such drug delivery devices can include medication pumps having a cannula that remains in the body to allow infusion over a multi-hour or multi-day period (e.g., wearable pumps for the delivery of basal and bolus insulin). Reader device 120, when combined with a medication pump, can include a reservoir to store the drug, a pump connectable to transfer tubing, and an infusion cannula. The pump can force the drug from the reservoir, through the tubing and into the diabetic's body by way of the cannula inserted therein. Other examples of drug delivery devices that can be included with (or integrated with) reader device 120 include portable injection devices that pierce the skin only for each delivery and are subsequently removed (e.g., insulin pens). A reader device 120, when combined with a portable injection device, can include an injection needle, a cartridge for carrying the drug, an interface for controlling the amount of drug to be delivered, and an actuator to cause injection to occur. The device can be used repeatedly until the drug is exhausted, at which point the combined device can be discarded, or the cartridge can be replaced with a new one, at which point the combined device can be reused repeatedly. The needle can be replaced after each injection.

The combined device may function as part of a closed-loop system (e.g., an artificial pancreas system requiring no user intervention to operate) or semi-closed loop system (e.g., an insulin loop system requiring seldom user intervention to operate, such as to confirm changes in dose). For example, the diabetic's analyte level can be monitored in a repeated automatic fashion by sensor control device 102, which may then communicate that monitored analyte level to reader device 120, and the appropriate drug dosage to control the diabetic's analyte level can be automatically determined and subsequently delivered to the diabetic's body. Software instructions for controlling the pump and the amount of insulin delivered may be stored in the memory of reader device 120 and executed by the reader device's processing circuitry. These instructions can also cause calculation of drug delivery amounts and durations (e.g., a bolus infusion and/or a basal infusion profile) based on the analyte level measurements obtained directly or indirectly from sensor control device 102. **In** some embodiments sensor control device 102 may determine the drug dosage and communicate that to reader device 120.

### Example Embodiments of Sensor Control Devices

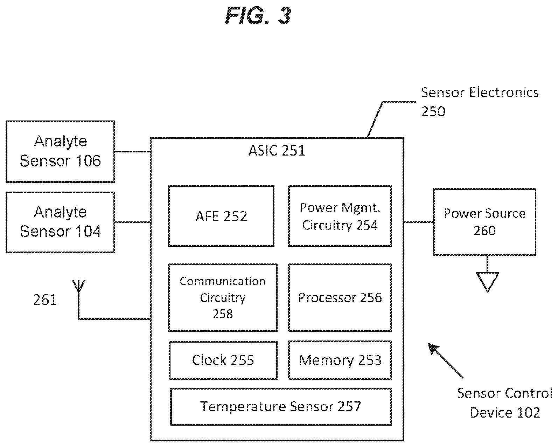

FIG. 3 is a block diagram depicting an example embodiment of sensor control device 102 having analyte sensor 104 and sensor electronics 250 (including analyte monitoring circuitry) that can have the majority of the processing capability for rendering end-result data suitable for display to the user. **In** FIG. 3, a single semiconductor chip 251 is depicted that may be a custom application specific integrated circuit (ASIC). Shown within ASIC 251 are certain high-level functional units, including an analog front end (AFE) 252, power management (or control) circuitry 254, processor 256, and communication circuitry 258 (which can be implemented as a transmitter, receiver, transceiver, passive circuit, or otherwise according to the communication protocol). **In** this embodiment, both AFE 252 and processor 256 are used as analyte monitoring circuitry, but in other embodiments either circuit can perform the analyte monitoring function. Processor 256 can include one or more processors, microprocessors, controllers, and/or microcontrollers, each of which can be a discrete chip or distributed among several different chips.

A memory 253 is also included within ASIC 251 and can be shared by the various functional units present within ASIC 251 or can be distributed amongst two or more processors. Memory 253 can also be a separate chip. Memory 253 is non-transitory and can be volatile and/or non-volatile memory. **In** this embodiment, ASIC 251 is coupled with power source 260, which can be a coin cell battery, or the like. AFE 252 interfaces with one or more in vivo analyte sensors 104, 106 and receives measurement data therefrom and outputs the data to processor 256 in digital form, which in turn can, in some embodiments, process in any of the manners described elsewhere herein. This data can then be provided to communication circuitry 258 for sending, by way of antenna 261, to reader device 120 (not shown), for example, where minimal further processing is needed by the resident software application to display the data. Antenna 261 can be configured according to the needs of the application and communication protocol. Antenna 261 can be, for example, a printed circuit board (PCB) trace antenna, a ceramic antenna, or a discrete metallic antenna. Antenna 261 can be configured as a monopole antenna, a dipole antenna, an F-type antenna, a loop antenna, and others.

Information may be communicated from sensor control device 102 to a second device (e.g., reader device 120) at the initiative of sensor control device 102 or reader device 120. For example, information can be communicated automatically and/or repeatedly (e.g., continuously) by sensor control device 102 when the analyte information is available, or according to a schedule (e.g., about every 1 minute, about every 5 minutes, about every 10 minutes, or the like), in which case the information can be stored or logged in a memory of sensor control device 102 for later communication. The information can be transmitted from sensor control device 102 in response to receipt of a request by the second device. This request can be an automated request, e.g., a request transmitted by the second device according to a schedule or can be a request generated at the initiative of a user (e.g., an ad hoc or manual request). **In** some embodiments, a manual request for data is referred to as a "scan" of sensor control device 102 or an "on-demand" data transfer from device 102. **In** some embodiments, the second device can transmit a polling signal or data packet to sensor control device 102, and device 102 can treat each poll (or polls occurring at certain time intervals) as a request for data and, if data is available, then can transmit such data to the second device. **In** many embodiments, the communication between sensor control device 102 and the second device are secure (e.g., encrypted and/or between authenticated devices), but in some embodiments the data can be transmitted from sensor control device 102 in an unsecured manner, e.g., as a broadcast to all listening devices in range.

Different types and/or forms and/or amounts of information may be sent as part of each communication including, but not limited to, one or more of current sensor measurements (e.g., the most recently obtained analyte level information temporally corresponding to the time the reading is initiated), rate of change of the measured metric over a predetermined time period, rate of the rate of change of the metric (acceleration in the rate of change), or historical metric information corresponding to metric information obtained prior to a given reading and stored in a memory of sensor control device 102.

Some or all of real time, historical, rate of change, rate of rate of change (such as acceleration or deceleration) information may be sent to reader device 120 in a given communication or transmission. **In** certain embodiments, the type and/or form and/or amount of information sent to reader device 120 may be preprogrammed and/or unchangeable (e.g., preset at manufacturing), or may not be preprogrammed and/or unchangeable so that it may be selectable and/or changeable in the field one or more times (e.g., by activating a switch of the system, etc.). Accordingly, in certain embodiments reader device 120 can output a current (real time) sensor-derived analyte value (e.g., in numerical format), a current rate of analyte change (e.g., in the form of an analyte rate indicator such as an arrow pointing in a direction to indicate the current rate), and analyte trend history data based on sensor readings acquired by and stored in memory of sensor control device 102 (e.g., in the form of a graphical trace). Additionally, an on-skin or sensor temperature reading or measurement may be collected by an optional temperature sensor 257. Those readings or measurements can be communicated (either individually or as an aggregated measurement over time) from sensor control device 102 to another device (e.g., reader 120). The temperature reading or measurement, however, may be used in conjunction with a software routine executed by reader device 120 to correct or compensate the analyte measurement output to the user, instead of or in addition to actually displaying the temperature measurement to the user.

**In** addition, although FIG. 3 depicts dual analyte sensors 104, 106, according to many embodiments of the present disclosure, sensor control device 102 can be configured to collect data indicative of multiple physiological measurements, including but not limited to, data indicative of a glucose level, lactate level, ketone level, or heart rate measurement, to name only a few. In some embodiments, for example, sensor 104 can be a dual-analyte sensor configured to sense a glucose level and a concentration of another analyte (e.g., lactate, ketone, etc.). Additional details regarding dual-analyte sensors are described, for example, in U.S. Publication No. 2019/0320947 A1, which is hereby incorporated by reference for all purposes. In some embodiments, sensor control device 102 can include multiple discrete sensors, each of which is capable of collecting data indicative of any of the aforementioned physiological measurements. For example, in some embodiments, a first analyte sensor 104 may be used to sense blood glucose, and a second analyte sensor 106 may be used to sense ketone.

### Embodiments of Systems, Devices and Methods for Combined Use of Blood Glucose and Ketone Data

T1 diabetes patients using SGLT-2i are at risk of experiencing euglycemic DKA. Discrete ketone test strips, along with CGM, are available but not practical for continuous monitoring of ketones. With the commercial availability of continuous ketone monitoring on the horizon, the ability to detect an adverse glucose/ketone condition in real-time will be available to patients. However, how the patient interprets these numbers and determines appropriate action is complex and foreign to these patients and will require the patient to contact an HCP for guidance. The present disclosure provides a system, method and apparatus for providing guidance regarding appropriate action to the patient at appropriate times, and for capturing additional information about the patient's condition at moments when the patient will remember it. As used herein, "real-time" means without delay except as required by technical constraints of the media in use.

Aspects of the system, method or apparatus may be embodied in a mobile application-based system that detects conditions where actions should be taken, provides guidance to the patient and provides a means to record important context concurrent with the condition that will be helpful for the HCP to know later when advising the patient how to avoid this condition in the future. The mobile application may be installed in a memory of a reader device and executed by one or more processors of the reader device.

The processing and functionality required for this system may all be contained in the patient mobile application, or some or part of it may be contained in a web server that supports the mobile application with processing, communication hub and reporting functionality. For brevity, the functionality described here will assume functionality on the mobile application or the server, noting that it can be in either place. References to the mobile application will inherently encompass both the application alone and the mobile application working in conjunction with a web server.

The application is configured to retrieve or receive glucose sensor data and ketone sensor data sampled by an on-body unit at regular intervals and provided to the application immediately after each sample is taken (i.e., "continuous" or "continuously"). The sampled analyte data may come from two separate sensors, or a dual analyte sensor where a single sensor provides data for both analytes. The system, method and apparatus described herein extends to a system that retrieves episodic discrete glucose measurements and/or discrete ketone measurements, or various combinations of discrete and continuous measurement. The technology may also support use of discrete measurements in situations where continuous measurements are not available.

The mobile application may retrieve or receive and process analyte sensor data from an on body unit (OBU) in near real-time, such as every minute, or 5 minutes or 10 or 15 minutes, for example, in synchrony with the OBU's sampling interval. The mobile application may perform real-time (RT) processing of the obtained sensor data at corresponding intervals. The sampling frequency of the glucose sensor may differ from the sampling frequency of the ketone sensor. In some embodiments, the obtaining the sensor data and/or real-time processing of the data may occur at different periods for different times of the day. For example, frequency of sampling and/or processing may depend on the likelihood that glucose levels will be high, to minimize unnecessary power consumption or communication bandwidth. For further example, real-time processing may be suspended or limited to a low periodicity during the early morning hours because of glucose-raising meals typically occur later during the day.

In an aspect, the mobile application may be configured to detect and respond to glucose + ketone conditions relevant to managing use of SGLT-2i use by T1 patients, or to any other use cases where high ketones are a concern.

In an example configuration, a system, method and apparatus may include default predetermined settings for condition thresholds (glucose and ketone), and output warning, alerts, and recommendations for action or treatment in textual, audible, graphical, or other output format. The mobile application may include user-configurable settings for thresholds and/or outputs, and may require the user or HCP to confirm the settings. In an aspect, the mobile application may permit adjustment of these settings via a setup menu.

Suitable default threshold settings may be, for example:
Moderate ketone threshold = 1.0 mmol/L
High ketone threshold = 3.0 mmol/L
Low glucose threshold = 70 mg/dL
High glucose threshold = 180 mg/dL
A table of thresholds may be extended to any positive integer number of thresholds with distinct text associated with each condition logic element defined by these thresholds.

Mobile application settings may include insulin dose amounts associated with different ketone ranges. The settings may be defined in insulin type, insulin units and/or percent Total Daily Dose (TDD) and TDD. Alternatively, associations between these settings may be defined as an equation with ketone value (and potentially other ketone-based metrics such as ketone rate-of-change), as an input and insulin dose or percent TDD as an output. Equation parameters may be included as part of the mobile application's configuration settings. For example, the percent insulin dose may be a function of ketone value, or the amount of insulin calculated may be subtracted up to a function of ketone value. Alternatively, instead of a function of ketone value, a different function using ranges of ketone values can be used, including using two or more ranges of ketone values as input to a function that modifies the insulin dose.

Configurable settings in the mobile application may also include an insulin sensitivity parameter, which the mobile application may include in determining a recommended insulin dose for reducing high glucose levels. The settings may further include carbohydrate amounts or carbohydrate ratios, which define the amount of insulin to cover a specific number of carbohydrates, or conversely, the amount of carbohydrates to cover a specific amount of insulin. These parameters, including Insulin Sensitivity (IS) and Carbohydrate Ratio (CR), may be defined as a range of parameters, where each element of this range is associated with a range of glucose and/or a range of ketones. This would accommodate, for example, when a patient's lower insulin sensitivity in the past day or more indirectly causes high ketone readings. These associations may be implemented as functions where glucose and/or ketones are the inputs, and insulin sensitivity and carbohydrate ratio are the outputs, and the equation parameters are part of the configuration settings.

The configuration settings may further include enabling an interface with a remote bolus calculator or dose guidance system, including an Automatic Insulin Delivery (AID) system.

The mobile application may further include a setting to indicate the patient's disease state and medication regimen, such that these features are enabled if the patient diabetes type is T1 and the patient is using SGLT-2i. The settings may also allow the mobile application to indicate whether the patient is using an insulin pump or using an AID system, or if the patient is on a carb-restricted diet. These indications may be used by the mobile application to alter the text that is associated with the condition logic or alter the condition logic itself.

Mobile Application User Interface (UI) logic: The mobile application may be configured to detect in real time when a glucose/ketone condition occurs according to condition states are determined by predetermined thresholds. The condition logic, and an example of the associated display text is shown below. When a condition is first realized, the mobile application may send a notification to the patient. For example, if ketones are detected to transition from below to above the high ketone threshold (or in an alternative if an initial ketone reading is above the high ketone threshold), the mobile application may cause a UI device to output:
Warning: "Your ketones are high!"
Recommendation: "Seek immediate medical attention!"

In an aspect, the mobile application UI may include an alert function, wherein an interactive UI object (e.g., a "button") is provided for the patient to acknowledge and silence the alert. If the alert is ignored, the mobile application may cause the alert to remain active for a short period, for example 15 seconds, and reactivate after a substantially longer period (e.g., every 15 minutes) until user input via the UI of the mobile application indicates that the user has acknowledged the alert.

The mobile application may cause a warning to continue to display whenever the user accesses the current ketone reading display screen in the UI. In addition, the mobile application UI may include an interactive UI object (e.g., a button) to output (e.g., display) a recommendation or supplemental information associated with the condition for which the warning has been output. In an alternative, or in addition, the mobile application may cause the recommendation or supplemental information located on a ketone display screen of the UI. Similar UI operation may be used to indicate all condition states and provide appropriate recommendation or supplemental information predetermined in a memory of the mobile application for each of the condition states. For example, the recommendation or supplemental information may further include recommended insulin and/or carbohydrate amounts for alleviating an over-threshold condition.

For further example, if the ketones transition from above a high threshold to below a low threshold, the mobile application may cause a UI device to output:
Notification: "Ketone levels have recovered"
Prompts: "Select from the list below the level of discomfort caused by high ketones" Further, the mobile application may cause a UI device to output a list of discomfort levels for the patient to select from, for example a list ranging from "No discomfort" and "Little discomfort", to "Admitted to emergency medical services - modest symptoms" and "Admitted to emergency medical services - severe symptoms". The mobile application will log the patient's selection. Once this information is entered and logged, the mobile application may cause the prompt to be discontinued until the next time the triggering condition is detected.

For further example, after detection of ketones above a high threshold or below a low threshold, the mobile application may cause a UI device to output a query, for example "What may have contributed to your high (or low, as the case may be) ketone experience? Select all that apply." The mobile application may output selection options via which the user can indicate a response to the query, for example, other illness, fasting, strenuous activity, insulin delivery pump fault, or AID insulin delivery suspension. The mobile application may log the patient's selection made via the UI. Once this information is entered and logged, the mobile application may discontinue the prompt until the next time the condition is entered. If the alert prompts have not been answered, the mobile application may reactivate the query at intervals, for example every hour, until detecting a user response to the query via the UI.

In an alternative embodiment, the mobile application may trigger an alert or query only if detected ketones have remained above a high threshold or below a low threshold for a defined period, for example 2 hours.

If ketones are moderate (between high and low thresholds) and glucose is above the high threshold, the mobile application may cause a UI device to output, for example:
Warning: "Ketone levels are moderately high and glucose is above target"
Recommendation: "If you haven't injected insulin within the last 3 hours, inject insulin to lower your glucose and stay hydrated. Check ketones and glucose every hour until you are back into the normal ketone range."
Prompt: "What may have contributed to your moderate ketone experience? Select all that apply."
Query response may be handled as described herein above. As before, if the mobile application does not detect a user response to the alert, it may activate the alert for a short period, for example 15 seconds, and reactivate after a substantially longer period, for example every hour, until a response is received or the detected condition changes.

If the ketones transition from above the moderate threshold (but not high), to below the low threshold, the mobile application may cause a UI device to output:
Notification: "Ketone levels have recovered"
Prompt: "Select from the list below the level of discomfort caused by moderate ketones"
Query response may be handled as described herein above.

If ketones are moderate (between high and low thresholds) and glucose is between the low and high thresholds, the mobile application may cause a UI device to output:
Warning: "Ketone levels are moderately high and glucose is in target" Recommendation: "If you haven't injected insulin within the last 3 hours, ingest 15 grams of carbs and inject sufficient insulin to cover these carbs. Stay hydrated.
Check ketones and glucose every hour until you are back into the normal ketone range."
Prompt: "What may have contributed to your moderate ketone experience? Select all that apply"
The mobile application may log the patient's selection made via the UI. Once this information is entered and logged, the mobile application may discontinue the prompt until the next time the condition is entered. As before, if the mobile application does not detect a user response to the alert, it may activate the alert for a short period, for example 15 seconds, and reactivate after a substantially longer period, for example every hour, until a response is received or the detected condition changes.

If ketones are moderate (between high and low thresholds) and glucose is below the low threshold, the mobile application may cause a UI device to output:
Warning: "Ketone levels are moderately high and glucose is low"
Recommendation: "Treat your low glucose. Stay hydrated. Check ketones and glucose every 15 minutes until your glucose is above 70 mg/dL."
Prompt: "What may have contributed to your moderate ketone experience? Select all that apply."
Query response may be handled as described herein above. If the mobile application does not detect a user response to the alert, it may activate the alert for a short period, for example 15 seconds, and reactivate after a substantially longer period, for example every twenty minutes, until a response is received or the detected condition changes.

An alternative embodiment for the situation where ketones are dropping from the high ketone state is as follows. If the ketones transition from above to below the high threshold, the mobile application may cause a UI device to output:
Warning: "Ketone levels are dropping"
"Time since ketones have been at the high level: X hours, Y minutes"
"Time since ketones have been at the moderate level: K hours, L minutes"

The mobile application may repeat the appropriate one of the foregoing alarm notifications periodically, for example, every hour, until the ketones remain below the moderate level for a reset period, for example, 4 hours. The reset period may be user-configurable in the settings of the mobile application.

The mobile application may automatically provide any of the forgoing ketone alerts, or a modified version of the alerts, to associated users connected via an auxiliary application, for example, a caregiver application. An electronic address of one or more linked caregiver applications may be set in a memory of the mobile application.

Ketone alerts and prompts are not limited to the foregoing examples. The mobile application may provide other prompts, for example, prompts for determining whether the user is ill, and if so, to discover details about the user's illness. This prompt information may be logged by the mobile application of a connected server, and may be designed to be useful for clinicians to determine the underlying cause of the high ketone episode. In an alternative, or in addition, if the mobile application is connected to an insulin delivery or insulin guidance system, the mobile application may determine automatically whether the patient is suffering from some form of insulin resistance, for example, if correction doses are not lowering glucose levels in an expected amount.

The mobile application may be configured to provide additional notification features when the mobile application is in communication with an insulin delivery pump or a connected insulin pen. For example, if the mobile application recommends an insulin dose, then after an appropriate waiting period, for example 15 minutes, if an insulin dose has not been delivered within the past 3 hours and 15 minutes and the prior analyte conditions are still detected, then the software will generate resend the notification perhaps with an additional indication that this is a repeated notification. This notification may repeat periodically, for example every 15 minutes, for as long as the conditions described have been met.

A default home screen generated by the mobile application for the UI may display or otherwise output glucose data, such as glucose value, rate of change and a glucose plot, reporting analyte levels detected by the OBU. The home screen may also indicate a ketone range detected by the OBU. In some embodiments, the home screen may omit any indication of ketone analyte levels if ketones are in are normal range or close to the baseline value. When ketones detected by the OBU are elevated, such as in the moderate or high range (or perhaps when sufficiently higher than baseline), the mobile application may cause the screen to display the current ketone value and optionally a rate of change, which may be calculated for various periods, for example, using 15 minutes of data, or 30 or 60 minutes of data. Also, the mobile application may cause the UI home screen to automatically replace the glucose time plot by the ketone plot. The ketone plot may use a different time range than the glucose plot, for example, 24 hours instead of 8 hours. Alternatively, the glucose and ketone traces may be collocated on one screen, for example glucose axis on the left and ketone axis on the right, and the time scale may switch to 24 hours. The screen may have a UI means to toggle information focus between glucose and ketones. When ketones are in the moderate or high range, the Mobile application may change the default home screen to be ketone centric rather than glucose centric. The home screen may show the ketone range, and indicate if there is low glucose.

The foregoing logic for UI interface control may be implemented as a state machine in the mobile application's executable memory.

The mobile application may further include treatment guidance, including insulin bolus calculation. For patient's that use a bolus calculator or a dose guidance system, the mobile application may provide specific recommendations for dosing and carbohydrate ingestion via its UI, for example using alerts as described above. The bolus calculator or dose guidance system (referred to herein as the "calculator," for simplicity) may be collocated on the mobile application and on a web server supporting the mobile application, or it may be located remotely from the mobile application. In the latter case, the remote calculator may provide an application program interface (API) that allows the mobile application to retrieve key parameters from the calculator, such as Insulin Sensitivity (IS) and Carbohydrate Ratio CR). The mobile application may then calculate the recommended insulin dose amount and carbohydrate amount for the patient to take, based on blood glucose and ketones detected by an OBU, and on other factors.

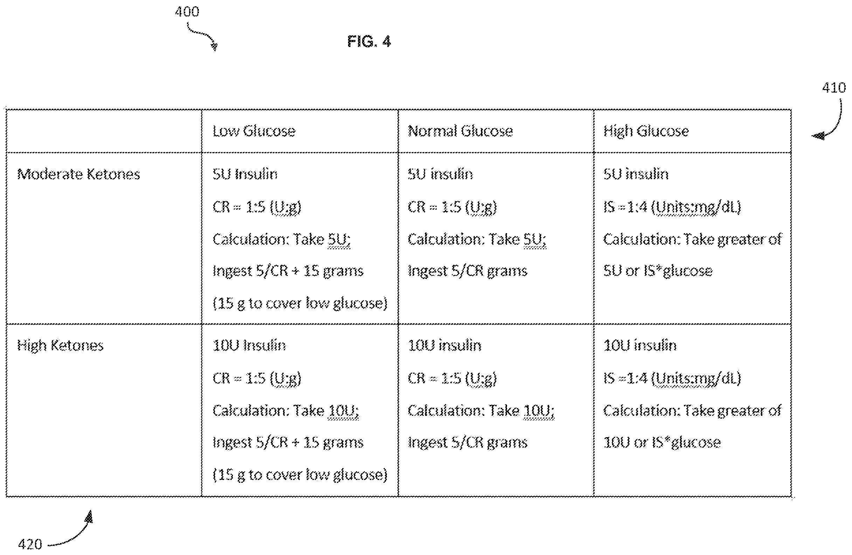

Table 400 shown in FIG. 4 illustrates one embodiment of treatment guidance parameters responsive to values of time-correlated glucose and ketone values from an OBU. Each of the columns 410 points out a different one of measured glucose states: low, normal or high. Each of the rows 420 points out a different one of measured ketone states: moderate or high. Data at each row-column intersection describes parameters for a therapeutic advisory message, mapping the conditional logic used for formulating different advice. Other data structures for mapping conditional logic may also be used. The mobile application may use this table, or another suitable mapping of conditional logic, to configure content of an advisory message for output by a reader device or other UI device.

Alternatively, the remote calculator may make these calculations and return the insulin dose and/or carbohydrate ingestion recommendation to the mobile application. The remote calculator may operate in accordance with a defined API via which the mobile application may request these outputs, provide the glucose level and ketone level (and potentially other derived metrics or time-series of level data), and manage other appropriate settings such as glucose and ketone thresholds, as inputs.

The conditional logic described herein and the corresponding user interface may be integrated into the user interface and logic for a) an insulin pump based AID system, or b) an insulin decision support system (MDI or pump). For both, the condition logic described may run concurrently with the AID or decision support functionality on the mobile application, and notifications may be issued asynchronously to the AID or decision support functionality, or they may be buffered and synchronized for display based on priority rules. It is likely that glucose condition states and ketone condition states may transition at different times, so for example, the mobile application may present a low glucose alert at one point in time, and a few minutes later the mobile application may present a moderate ketone and low glucose alert. The mobile application software may be designed to modify the behavior of alerts presented; in the example above, the low alert behavior after its initial notification may be altered or suppressed in favor of the moderate ketone and low glucose alert post-notification behavior.

In another scenario, the glucose ketone consideration logic may provide a button that displays the appropriate meal-time dose guidance, along with the other recommendations. So if the recommendation is for the patient to ingest carbs and cover with insulin, for example, and the current time is around lunch-time, the mobile application may provide a button labeled "lunch", when depressed will display the dose guidance for lunch.

If the glucose ketone condition logic is integrated with an AID system, the condition logic may prevent the AID system from fully suspending insulin delivery when glucose is low or predicted to be low.

The dose guidance determined by a bolus calculator may be transmitted to the patient's insulin pump or AID system via an API. The mobile application UI may provide a UI method for the patient to confirm their intention to initiate the insulin dose and consume the appropriate amount of carbohydrates, if needed.

Traditional insulin bolus calculators estimate the amount of insulin needed to either a) reduce a patient's glucose to a desired level, and/or b) to compensate for an anticipated meal. These calculators commonly take into account current glucose level, desired glucose level, estimated insulin-on-board, and estimated anticipated carbohydrate intake, based on a mathematical model of the patient's glycemic response to insulin and carbohydrates.

Traditional bolus calculators assume that the patient's glycemic response is constant or has different values during the day. The glycemic response is typically characterized for a patient by the patient's individual "basal insulin", "insulin sensitivity" and "carbohydrate ratio" factors. Basal insulin is the amount of long acting insulin taken during the day (or in the case of insulin pump users, the amount of rapid acting insulin provided continuously). Insulin sensitivity (IS) is the estimated amount of insulin required to lower 1 unit of glucose. Carbohydrate ratio (CR) is the amount of insulin required to compensate for 1 unit of carbohydrate intake. Thus,
Basal = constant (units per day);
IS = constant (mg/dL per unit); and
CR = constant (CHO per unit).

It is well known that this simple glycemic model is not always accurate, and conditions such as activity level and alcohol consumption may cause the patient's actual physiology to deviate from the simple model. Actual glycemic response is more complex than what can be described by these three factors. However, more complex glycemic response models are not well understood and are more difficult to define for a patient. Use of glycemic response models that use these three factors represents the present standard of care for insulin using patients.

The mobile application may include novel enhancements to present insulin calculators. Specifically, the glycemic response model that defines how the insulin calculator works may be enhanced by utilizing one or more additional analyte measurements or additional information. Other potential analyte measurements that could help make the glycemic response model more accurate may include current ketone level and current trend in ketone level, current lactic acid level, fat content in the user's next anticipated meal, the user's anticipated activity level after the meal, and the user's activity level in the period prior to the meal for which the bolus is calculated.

Glycemic response is impacted by meal composition and activity level. The mobile application may include enhancements to the bolus calculator based on additional analyte measurements. To illustrate, two potential bolus calculators are described, one using ketone measurements and one using lactic acid measurements.

With regard to glycemic response, persistently elevated ketones may be an indicator of reduced insulin sensitivity, increased carbohydrate ratio, and requiring more basal insulin. Consequently, when ketones are higher than normal, more insulin is required in order to achieve the same effect on glucose. The model used for the bolus calculator of a mobile application may, in one embodiment, be modified to account for this effect by replacing these constant factors by functions based on ketone level. In the simplest form, the functions may be based on the current ketone reading. $Basal = constant * f \left(Ketones\right) \left(units per day\right)$ $IS = constant * f \left(Ketones\right) \left(mg/dL per unit\right)$ $CR = constant * f \left(Ketones\right) \left(CHO per unit\right)$ An example of a simple step-wise linear model is as follows:
If ketones < 1.0 mmol/L
   Then IS = 4 mg/dL/Unit (or in common notation, 1:4)
If ketones >= 1.0 AND < 3.0 mmol/L,
   Then IS = 4 + {ketone - 1)/2} * 4 mmol/L
Otherwise ketones = 8 mmol/L

A more advanced bolus calculator may use functions that are based on more than one ketone measurements. As noted herein, ketones can be measured with frequent periodicity (for example, every 15 minutes) using a continuous sensor. The benefit of continuous data is that a more accurate glycemic response model that utilizes continuous ketone data may be possible which could account for dynamic effects of ketones on glycemic response.

Other models can be contemplated generally where the ketone measurement is incorporated. For example, rather than modeling ketones as a parameter of the model, ketones, and higher order derivatives of ketones, can be modeled as state variables along with glucose and its higher order derivatives.

In an alternative, or in addition, the mobile application may be programmed to perform an even more advanced bolus calculator using model-based fitted parameters and trained with data from a similar patient population. An adaptive model may also be applied where the mobile application begins with a populated-trained model by default, and over time adapts using the patient's data to further train the model. For example, the mobile application may track prior insulin dose amounts paired with glucose measurement -derived data (such as glucose values at specific time points relative to the insulin dose, glucose rate of change values at specific time points relative to the insulin dose, area under the curve of glucose above a pre-determined threshold for a pre-determined time relative to the insulin dose, and others), and ketone measurement-derived data. The mobile application may incorporate a pre-determined model calculated to estimate an effect of data prior to the insulin dose on data after the insulin dose. Over time, the mobile application, or a separate heuristic application, may update key parameters of the model using recursive estimation methods, parameter regression methods, neural networks, or other heuristic method. Alternatively, data prior to the insulin dose can be used to build features that predict certain attributes associated with the user's historical data after the insulin dose, for every insulin dosing event in the past. The model development algorithm may provide the historical data to one or more machine learning frameworks to develop an estimator that can update the dosing parameters by accounting for both glucose and ketone -derived data up to every insulin dosing decision moving forward. The resulting bolus calculator may have different set of equations under different conditions determined by the glucose and/or ketone - derived data, where machine learning framework, for example, random forest, may be able to provide the condition thresholds and the equations that determine the insulin dose.

An OBE in communication with the mobile application may measure lactic acid frequent periodicity using a continuous sensor. High lactic acid is associated with a high level of activity and may be useful as an automatic measure of activity. High lactic acid tends to make glucose reduction more responsive to insulin than normal. Lactic acid measurements can be incorporated into the bolus calculator model, similarly as described for ketones.

The mobile application may incorporate modeling for AID systems in which insulin is automatically delivered to patient. Here, the AID model may include ketone and lactate measurements as inputs. Alternatively, the AID system may include multiple models, where the ketone and lactate measurements define which model to use. For example, one model may utilize a lower insulin sensitivity than another; if ketones are above a particular threshold, then the system may switch to use the model that utilizes a lower insulin sensitivity.

The mobile application may include code for using ketone measurement to detect when a user/patient is sick. When the mobile application detects elevated ketones, it may prompt the user to indicate whether they are unwell. If the patient indicates that they are unwell, the mobile application may alter parameters of a bolus calculation model, or switch to a different model that is more appropriate for when the patient is ill.

In general, the mobile application may make use of a bolus calculator based on a model that simultaneously takes into account any number of additional analyte measurements that may better describe a patient's glycemic response.

The mobile application may be configured for providing guidance for insulin pump wearers or AID users. If the mobile application has been configured for a pump wearer, the recommendations output via a UI may include instructions to check the proper operation of the pump to ensure that insulin is being delivered. For AID uses, the AID system may suspend insulin delivery, so a recommendation may be to manually override the AID system to restart insulin delivery. The mobile application may electronically connect with the AID system and automatically restart insulin delivery, while recommending that the patient consume carbohydrates as part of the treatment.

The mobile application may be configured for providing guidance for those on carbohydrate restricted diets. For these users/patients, the clinician may need to increase the moderate ketone level threshold configuration parameter of the mobile application to accommodate. In an alternative, the mobile application may automatically adjust this level if the carbohydrate restricted diet option is selected, based on its configuration. The mobile application may conduct periodic data processing to determine whether the patient can maintain moderately high ketones without causing high ketones, and automatically set the moderate threshold or make a recommendation in a report output to the HCP how to adjust this threshold. Additional information obtained periodically from the patient to assess their beta cell function, such as their C-peptide levels, may be used to determine the relative risk of ketoacidosis versus diet ketosis. For example, one with a lower beta cell function will have a higher relative risk of ketoacidosis, and thus should not choose a raised ketone threshold level.

The mobile application may be configured for providing guidance for SGLT-2i users for patients using SGLT-2i or similar acting medication, or specifically for T1 patients using SGLT-2i. In this configuration, the mobile application may make the recommendations for moderate or high ketones including instructions to discontinue use of SGLT-2i until instructed by their HCP to start again.

The mobile application may be configured for providing reporting to a patient and/or HCP. The mobile application may report ketones using daily time traces much like glucose daily traces are commonly reported today. The mobile application may include a ketone trace with the glucose trace on the same report. In an embodiment, information gathered from the prompts displayed by the mobile application may be used to annotate these traces, where a symbol representing the cause of the high ketone episode (or the text of the cause itself) may be located vertically coincident with the time it was logged or with the time that the ketones started increasing. For example, if the cause was indicated to be due to pump failure, then an icon related to pump failure can be placed at the start of the associated ketone increase. Likewise, discomfort level info logs may be annotated on the trace, placed at the start of the associated high ketone event, or at the end or in the middle.

Alternatively, the mobile application may annotate a glucose time-trace plot with colored bands or other such indicators of ranges of ketones; for example, a green band indicating the normal ketone range, a yellow band indicating the moderate ketone range, and a red band indicating the high ketone range.

The mobile application may report other metrics for providing medical insight into the patient's condition, for example frequency (e.g., occurrences per year) of high ketone events, moderate ketone events, high ketone events occurring with normal target range glucose (all glucose and ketone range possibilities), high ketone causes, and ketone baseline level. For the high ketone events over the past year, the mobile application may collect and report frequency of causes and the distribution of discomfort level reported by the patient. In addition, or in an alternative, the mobile application may report the relationship between moderate ketone levels and high ketone levels, for example, how often high ketone events occur if there is a moderate ketone event. A mobile application may provide a modal day plot of ketones to indicate whether there is a time-of-day prevalence for high ketones. A modal week plot may serve the same purpose to identify days of the week that may be at higher risk, and to help tease out with the patient what factors may be causing the high ketone episodes. The report may identify and display an indication that SGLT-2i use or carbohydrate restricted diets are not advised for a particular patient, based on these metrics; for example, if a patient has 2 or more occurrences of high ketones in the past year.

The mobile application may collect, process and report discomfort levels reported by the patient, and associate the discomfort levels with treatment actions to illustrate the benefits of treatment. For example, the mobile application may calculate and output a metric configured to show that if recommended treatments were followed, then high ketone episodes were prevented, or that high ketone episodes resulted in lesser discomfort levels. For example, the report may present the frequency of high ketone events when treatment was followed, compare to the frequency when treatment was not followed vs was delayed, and it may present the distribution of discomfort levels when high ketone events occur, when treatment recommendations were followed compared to when recommendations were not followed or when compliance is delayed.

The mobile application may feed output information from such ketone and treatment analysis back into a ketone threshold determination. For example, an analysis may determine that, for a particular patient, ketones up to the level of 1.4 do not transition into high ketones, but some ketones above this value do, indicating that the low ketone threshold may be set to 1.4. This process could be performed periodically by the mobile application and reported to the HCP (for manual adjustment of the threshold), or automatically adjusted by the mobile application.

The mobile application may include in output reports time traces of individual moderate and high ketone events, annotated or aligned with other information such as meal logs, insulin deliver logs or records and glucose traces, and other possible data to illustrate the effectiveness of the treatment to mitigate high ketones. The mobile application may plot continuous ketones levels using modal day plots, with individual ketone traces or with percentile traces, for example 5%, 25%, 50%, 75% and 95%-iles calculated for each hour of the day. The mobile application may produce and chronologically align overlay plots to key events on the ketone trace, such as the time when ketones start increasing or when ketones cross a certain threshold.

The mobile application may be configured for reporting data that can be used for a diabetes type diagnosis by an HCP, based on a marker for measuring C-peptide (beta cell function). The mobile application may perform periodic advanced analyses. For example, the mobile application may perform an assessment of the baseline ketone level for the patient by calculating a median ketone level over one or more periods, assuming that most of the time the patient is at a baseline level. Other known techniques for determine a baseline value may also be performed.

The mobile application may use a predictive model based on the data inputs and result outputs described here, where the likelihood of a high ketone event may be estimated and presented to the patient. The mobile application may present this on demand, perhaps as part of the ketone measurement screen, or may present as a notification when a condition is detected, such as 50% change of a high ketone event. The predictive model used by the mobile application may be developed using standard modeling techniques using data from a population of patients where the inputs and outputs described are retrieved. The model may be adaptive to the individual user/patient when sufficient input/output data from that patient are retrieved.

For example, the mobile application may use a predictive model derived from a population of patients is used in the beginning of a patient's sensor wear. Over time, if there are certain parameters that modulate the predictive model into a range of known variations from the population model, the mobile application, or a separate modeling application in communication with the mobile application, may estimate and update the parameters over time. With the updated parameter, the predictive model will be able to better estimate the patient's near future ketone readings. Methods using standard regression techniques, model-based parameter adaptation, supervised machine learning, or unsupervised machine learning, can be used. Alternatively, if the predictive model is intended to present a small, quantized set of value ranges, a classification-based approach can be used.

The mobile application may incorporate rate of change ("ROC") into the condition logic. Condition range definitions may additionally depend on ketone (or glucose) rate-of-change (ROC). For example, the moderate range may be defined by ketones > 1 mmol/L OR (ketones > 0.5 mmol/L AND ketoneROC > 0.3 mmol/L/hr), where the ketoneROC can be estimated a variety of ways including simply determining the slope the most recent 15 minute window of ketone data. The condition logic may also incorporate hysteresis; that is, if the condition is determined to be moderate ketones based on the ROC logic, to return to the normal ketone range, the condition logic may need to be (ketones < 1.0 mmol/L AND ketoneROC < 0 mmol/L/hr). This is to prevent erratic condition changes due to noisy rate of change estimation.

If other sensor data are collected by the mobile application, such as activity data or heart rate or another analyte sensor data that may help predict high ketone occurrences, a probabilistic model can be made between these data and occurrences of high ketone levels, and the model can be used to predict when high ketones may be likely and an appropriate alert is sent to the patient, for example as further described herein above.

In another aspect, the mobile application may automatically titrate the SGLT-2i dose amount for the patient. The mobile application may base the titration on ketone levels, and other analyte levels, such as glucose, and other measurements, such as delivered insulin or carbohydrate intake. In one embodiment, the mobile application may process glucose and ketone levels periodically, such as once per month, to determine if the SGLT-2i dose amount should be increase, decreased or left the same. The mobile application may output any change or hold recommendation to the patient by the mobile application UI. Optionally, the mobile application may send the recommendation to an HCP for approval prior to output by the mobile application to the patient. Alternatively, the reporting software may provide a UI procedure to initiate a report that initiates the periodic processing and outputs the result in the report.

In embodiments, the mobile application may retrieve a most recent period (e.g., two weeks) of glucose and ketone data. The mobile application may calculate a glucose variability metric, for example, a standard deviation of the entire glucose data, or just the day-time period, or a glucose-time area metric associated with the 5-hour period following each meal. The mobile application may further calculate a median ketone level, filtering out any moderate or high ketone data, to establish a baseline ketone level. For example, the mobile application may output a recommendation for an increase in the SGLT-2i dose amount if a) the glucose variability is greater than a predetermined threshold, b) the baseline ketone is less than a predetermined threshold, c) no high ketone values were recorded over the past year, and d) no moderate ketone values associated with concurrent glucose values less than a predetermined threshold were recorded over the past year. For further example, the mobile application may output a recommendation for a decrease in the SGLT-2i dose if a) the ketone baseline exceeds a predetermined threshold, or b) if any high ketone values were recorded over the past year. Otherwise, no change is recommended. The mobile application may set default dose amounts equal to amounts typically prescribed by clinicians, provide a UI means to configure these increments, or both. The titration logic criteria may also be configurable.

The mobile application may use data from a ketone sensor to detect faults in an OBU glucose sensor, and vice versa. For systems where the mobile application is connected to the insulin pump, or connected pen, the insulin delivery data may be useful as well. If the ketones are high, but the glucose levels are low or normal, this could be an indication of euglycemic DKA or it could be an indication that the glucose sensor is erroneously reading low, and the patients glucose levels are actually high. Accordingly, if the foregoing conditions are detected, the mobile application may cause output recommendations to include instructions to confirm the glucose sensor reading with a blood glucose test strip, or other redundant means of glucose measurement. If insulin delivery data are available, and a recent insulin delivery event occurred, then this may indicate that the sensor is functioning properly. In an embodiment, the mobile application may use a glucose prediction model based on insulin delivery data and/or insulin dose guidance information, for example, information concerning whether the patient sought guidance for a prandial insulin dose, or a high-glucose-correction dose. If the measured glucose is substantially different than the predicted glucose, this may be an indication of a glucose sensor fault. Likewise, high ketones and low/normal glucose may be an indication of a ketone sensor fault, and the recommendation by the mobile application may include instructions to redundantly check ketones with a blood ketone test strip.

Alternatively, if glucose reading are high and ketone reading are low, this again may be an indication of sensor error. The recommendation by the mobile application may be to do a redundant measurement to confirm the sensor function. If the mobile application detects that insulin was recently delivered, this may point to a glucose sensor fault; otherwise, this may point to a ketone sensor fault.

The glucose ketone sensor(s) may be used to detect issues with pump delivery of insulin, such as infusion set occlusion, or issues with pen delivery of insulin. If ketones are high and glucose levels are high, and insulin delivery was recorded, this may be an indication of a fault with the insulin delivery where some or all of the insulin was not delivered to the patient. If the mobile application detects these conditions, it may output a recommendation and instructions for checking the function of the insulin delivery system.

Another indication of a pump occlusion can be that the system detects that ketones are rising faster than glucose. For example, if the mobile application detects that the ketones rate-of-change has exceeded a predetermined threshold, followed by the glucose exceeding a predetermined rate-of-change threshold, it may output a recommendation and instructions for checking the function of the insulin delivery system.

High ketones concurrent with low glucose may also be an indication of an anomalous sensor attenuation, which may occur at the beginning of the sensor wear, or late in the sensor wear or during the sensor wear when the patient may be applying pressure to the sensor when lying down. In an AID system (or similarly with a manually injected insulin system), if the sensor is reading low, then insulin delivery may be low or suspended. Glucose levels may be rising undetected while ketone levels are rising and are detected. If the mobile application detects these conditions, it may cause the high ketone alert output to indicate to the patient to confirm their glucose level with a blood glucose test strip.

Detection of these anomalous events and sensor errors may be performed retrospectively. An elevated ketone level concurrent with low insulin delivery data when detected may cause the report generation process to exclude from the report calculations the glucose data prior to (for example 4 hours) and up to the point when the detection condition is no longer indicated.

In another embodiment, the mobile application may incorporate a pump-occlusion detection subsystem, for example a method for measuring the tubing pressure during insulin delivery. If the pressure exceeds a threshold, then the mobile application may output a notification to the patient of a possible occlusion, via its UI. The mobile application may use the occlusion detection method described above using glucose and ketone measurements to augment this system. When the glucose and ketone conditions indicate a possible occlusion, the mobile application may adjust the parameters of its occlusion detection system, for example, by lowering the pressure threshold used to detect occlusion. The parameters and outputs of both the glucose/ketone occlusion detector and the other (e.g. pressure) occlusion detector may be integrated in any number of ways to provide a more reliable occlusion detection method and outputs.

### Method For Combined Use of BG and Ketone Data

Example embodiments of methods for a dual analyte sensing using glucose history from a glucose sensor in combination with data from a ketone sensor for controlling a user interface device, health care provider interface device, or dose administration device for improved control of a patient's glucose level will now be described. Before doing so, it will be understood by those of skill in the art that any one or more of the steps of the example methods described herein can be stored as software instructions in a non-transitory memory of a sensor control device, a reader device, a remote computer, or a trusted computer system, such as those described with respect to FIG. 1. The stored instructions, when executed, can cause the processing circuitry of the associated device or computing system to perform any one or more of the steps of the example methods described herein. It will also be understood by those of skill in the art that, in many of the embodiments, any one or more of the method steps described herein can be performed using real-time or near real-time sensor data. In other embodiments, any one or more of the method steps can be performed retrospectively with respect to stored sensor data, including sensor data from prior sensor wears by the same user. In some embodiments, the method steps described herein can be performed periodically, according to a predetermined schedule, and/or in batches of retrospective processes.

It will also be appreciated by those of skill in the art that the instructions can be stored in non-transitory memory on a single device (e.g., a sensor control device or a reader device) or, in the alternative, can be distributed across multiple discrete devices, which can be located in geographically dispersed locations (e.g., a cloud platform). For example, in some embodiments, the collection of data indicative of an analyte level (e.g., glucose, ketone) can be performed on the sensor control device, whereas the calculation of analyte metrics (e.g., glucose derivative values, ketone derivative values) and the comparison of said analyte metrics to predetermined thresholds can be performed on a reader device, remote computing system, or a trusted computing system. In some embodiments, the collection of analyte data and comparison of with predetermined thresholds can be performed solely on the sensor control device. Likewise, those of skill in the art will recognize that the representations of computing devices in the embodiments disclosed herein, such as those shown in FIG. 1, are intended to cover both physical devices and virtual devices (or "virtual machines").

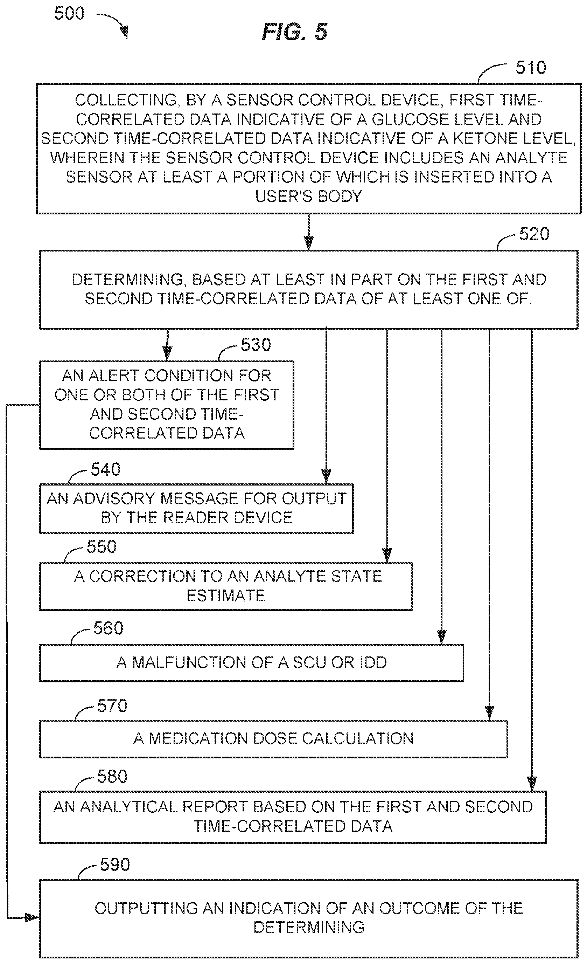

FIG. 5 is a flow diagram of an example embodiment of a method 500 for analyte monitoring and control in a patient. At Step 510, a first data indicative of a glucose level is collected by an analyte sensor, such as those described with respect to FIGS. 1 and 3. Also at 510, a second data indicative of a ketone level is collected by a ketone sensing element. According to some embodiments, Step 510 can be performed by a sensor control unit (SCU, also called an on body unit) comprising an analyte sensor having a portion that is configured to be inserted into a user's (i.e., the patient's) body at an insertion site, wherein the portion includes a first sensing element configured to sense a glucose level in a bodily fluid and a second sensing element configured to sense a ketone level in the bodily fluid of the same insertion site. In other embodiments, Step 510 can be performed by a sensor control unit comprising a first analyte sensor and a second analyte sensor, wherein the first analyte sensor is configured to sense a glucose level in a bodily fluid and the second analyte sensor is configured to sense a ketone level in the bodily fluid, and wherein the first and second analyte sensors are configured to sense analyte levels at the same localized site of insertion.

At 520, at least one processor of an SCU/OBU, a reader device communicatively coupled to the SCU, a server communicatively coupled to the SCU and/or reader device, or any useful combination of the foregoing, performs a determination of at least one output selected from the output represented by boxes 530-580. Each of these separate determinations 530-580 may be as described in more detail herein above. The at least one processor may perform any one or more of the determinations 530-580, in any useful order, not limited to the order listed in FIG. 5.

At 530, the at least one processor determines whether a condition for triggering output of an alert has occurred. The alert may concern the time-correlated glucose data, the ketone data, or both. Any of the conditions and alerts described in the foregoing disclosure may be used, and similar conditions and alerts.

At 540, the at least one processor determines content of an advisory message for output to a user interface of the reader device or other device. Any one of the advisory messages and questions for the user ("queries") described herein above may be determined, and similar messages or questions. Many non-limiting examples of conditional logic for determining advisory messages or questions are presented in the foregoing disclosure. The method 500 is not limited to specific examples given above. For example, parameters of the conditions, and content of the messages or questions may vary and be further developed to improve effectiveness and useability.

At 550, the at least one processor determines a correction to an analyte state estimate, for example, a blood glucose level or a ketone level, at a current time or at one or more past times, or a projected analyte level at a future time. The at least one processor may use any one or more of the corrective algorithms disclosed herein above to correct an analyte state estimate.

Similarly, at 560, the at least one processor may determine a likelihood whether the sensor control unit or an insulin delivery device (IDD) is malfunctioning, based at least in part on the time-correlated glucose and ketone data. Examples of logic for such determinations is provided in the detailed description above.

At 570, the at least one processor calculates a medication dose, for example, an insulin bolus dose, based at least in part on the time-correlated glucose and ketone data. Examples of dose calculation algorithms based on glucose and ketone data are provided in the foregoing disclosure.

At 580, the at least one processor determines content for an analytical report, based at least in part on the time-correlated glucose and ketone data.

At step 590, the at least one processor performs outputting the output determined at step 520, including any one or more of the determinations 530-580. The outputting may include formatting digital data for output by a display screen driven by a graphical user interface, or for audible output, for example. Thus, the systems and apparatus as described herein may perform the method 500 for control of insulin dosing and control of a user interface device for guiding a user to better insulin control.

For each and every embodiment of a method disclosed herein, systems and devices capable of performing each of those embodiments are covered within the scope of the present disclosure. For example, embodiments of sensor control devices are disclosed and these devices can have one or more analyte sensors, analyte monitoring circuits (e.g., an analog circuit), memories (e.g., for storing instructions), power sources, communication circuits, transmitters, receivers, clocks, counters, times, temperature sensors, processors (e.g., for executing instructions) that can perform any and all method steps or facilitate the execution of any and all method steps. These sensor control device embodiments can be used and can be capable of use to implement those steps performed by a sensor control device from any and all of the methods described herein. Similarly, embodiments of reader devices are disclosed and these devices can have one or more memories (e.g., for storing instructions), power sources, communication circuits, transmitters, receivers, clocks, counters, times, and processors (e.g., for executing instructions) that can perform any and all method steps or facilitate the execution of any and all method steps. These reader device embodiments can be used and can be capable of use to implement those steps performed by a reader device from any and all of the methods described herein. Embodiments of computer devices and servers are disclosed and these devices can have one or more memories (e.g., for storing instructions), power sources, communication circuits, transmitters, receivers, clocks, counters, times, and processors (e.g., for executing instructions) that can perform any and all method steps or facilitate the execution of any and all method steps. These reader device embodiments can be used and can be capable of use to implement those steps performed by a reader device from any and all of the methods described herein.

Computer program instructions for carrying out operations in accordance with the described subject matter may be written in any combination of one or more programming languages, including an object oriented programming language such as Java, JavaScript, Smalltalk, C++, C#, Transact-SQL, XML, PHP or the like and conventional procedural programming languages, such as the "C" programming language or similar programming languages. The program instructions may execute entirely on the user's computing device, partly on the user's computing device, as a stand-alone software package, partly on the user's computing device and partly on a remote computing device or entirely on the remote computing device or server. In the latter scenario, the remote computing device may be connected to the user's computing device through any type of network, including a local area network (LAN) or a wide area network (WAN), or the connection may be made to an external computer (for example, through the Internet using an Internet Service Provider).

It should be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the foregoing description does not explicitly state, in a particular instance, that such combinations or substitutions are possible. It is explicitly acknowledged that express recitation of every possible combination and substitution is overly burdensome, especially given that the permissibility of each and every such combination and substitution will be readily recognized by those of ordinary skill in the art. Preferred features are set out in the dependent claims and may be implemented in combination together with each of the aspects set out in the independent claims. Apparatus comprising means for implementing each of the methods are also provided.

To the extent the embodiments disclosed herein include or operate in association with memory, storage, and/or computer readable media, then that memory, storage, and/or computer readable media are non-transitory. Accordingly, to the extent that memory, storage, and/or computer readable media are covered by one or more claims, then that memory, storage, and/or computer readable media is only non-transitory.

As used herein and in the appended claims, the singular forms "a," "an," and "the" include plural referents unless the context clearly dictates otherwise.

While the embodiments are susceptible to various modifications and alternative forms, specific examples thereof have been shown in the drawings and are herein described in detail. **It** should be understood, however, that these embodiments are not to be limited to the particular form disclosed, but to the contrary, these embodiments are to cover all modifications, equivalents, and alternatives falling within the spirit of the disclosure. Furthermore, any features, functions, steps, or elements of the embodiments may be recited in or added to the claims, as well as negative limitations that define the inventive scope of the claims by features, functions, steps, or elements that are not within that scope.

### Clauses

Exemplary embodiments are set out in the following numbered clauses.
1. An analyte monitoring system, comprising:
   a sensor control device including an analyte sensor, first processing circuitry, and a first non-transitory memory, wherein the analyte sensor includes at least a portion configured to be inserted into a user's body, and wherein the sensor control device is configured to collect first time-correlated data indicative of a glucose level and second time-correlated data indicative of a ketone level; and
   a reader device comprising second processing circuitry and a second non-transitory memory,
   wherein at least one of the first or the second non-transitory memory includes instructions that, when executed, cause at least one of the first or the second processing circuitry to perform:
      determining, based at least in part on the first and second time-correlated data, at least one of:
      an alert condition for one or both of the first and second time-correlated data, an advisory message for output by the reader device, a correction to an analyte state estimate, a malfunction of the sensor control device, a malfunction of an insulin delivery device, a medication dose calculation, or an analytical report based on the first and second time-correlated data; and
      outputting, by the reader device, an indication of an outcome of the determining.
2. The analyte monitoring system of clause 1, wherein the instructions for determining the alert condition include instructions for setting at least one of a threshold of analyte level for the alert condition or an alert enunciation time interval.
3. The analyte monitoring system of clause 1 or 2, wherein the instructions for determining the advisory message include instructions for outputting a query by the reader device.
4. The analyte monitoring system of clause 3, wherein the instructions for outputting the query include instructions for outputting at least one question about a comfort level of a patient, based on the second time-correlated data indicating a transition from above a high threshold to a low threshold.
5. The analyte monitoring system of clause 3 or 4, wherein the instructions for outputting the query include instructions for outputting at least one question about prior patient behavior.
6. The analyte monitoring system of any preceding clause, further comprising an input device operatively coupled to at least one of the reader device or the sensor control device configured for receiving information defining insulin dosing by a patient wearing the sensor control device, wherein the instructions for determining include instructions for correcting an estimate of the patient's plasma insulin state.
7. The analyte monitoring system of any preceding clause, further comprising an input device operatively coupled to at least one of the reader device or the sensor control device configured for receiving a ketone test result, wherein the instructions further cause automatic execution of an insulin dose calculation algorithm in response to receiving the ketone test result.
8. The analyte monitoring system of any preceding clause, wherein the instructions for making the determining include instructions for applying conditional logic defined in a data structure to determine content of the advisory message.
9. The analyte monitoring system of any preceding clause, wherein the medication dose calculation comprises an insulin dose calculation.
10. The analyte monitoring system of clause 9, wherein the instructions for determining include instructions for the insulin dose calculation based on a current ketone level.
11. The analyte monitoring system of clause 9 or 10, wherein the instructions for determining include instructions for the insulin dose calculation comprising a mealtime bolus dose.
12. The analyte monitoring system of clause 9, 10 or **11,** wherein the instructions for determining include instructions for the insulin dose calculation using an adaptive model trained with data from a patient population.
13. The analyte monitoring system of any of clauses 9 to 12, wherein the instructions for determining include instructions for the insulin dose calculation further based on sensor data indicating a lactic acid measure.
14. The analyte monitoring system of any preceding clause, wherein the instructions for determining the advisory message include instructions for performing the determining further based on whether a patient is dosing with SGLT-2i, and for configuring the advisory message to discontinue use of SGLT-2i if ketones are above a threshold.
15. The analyte monitoring system of any preceding clause, wherein the instructions are stored on the second non-transitory memory.
16. The analyte monitoring system of any of clauses 1 to 14, wherein the instructions are stored on the first non-transitory memory.
17. The analyte monitoring system of any preceding clause, wherein the sensor control device further includes wireless communications circuitry configured to transmit the first and the second data to the reader device.
18. The analyte monitoring system of clause 16, wherein the wireless communications circuitry is configured to transmit the first and the second data according to a Bluetooth protocol.
19. The analyte monitoring system of any preceding clause, further comprising a third analyte sensor configured to sense a lactic acid level in the bodily fluid.
20. The analyte monitoring system of any preceding clause, further comprising a medication delivery device.
21. The analyte monitoring system of clause 19, wherein the medication delivery device comprises an insulin pump.
22. A computer-implemented method for analyte monitoring and control for a patient, the method comprising:
   collecting, by a sensor control device, first time-correlated data indicative of a glucose level and second time-correlated data indicative of a ketone level, wherein the sensor control device includes an analyte sensor at least a portion of which is inserted into a user's body;
   determining, based at least in part on the first and second time-correlated data, at least one of:
      an alert condition for one or both of the first and second time-correlated data, an advisory message for output by the reader device, a correction to an analyte state estimate, a malfunction of the sensor control device, a malfunction of an insulin delivery device, a medication dose calculation, or an analytical report based on the first and second time-correlated data; and
      outputting, by a user interface device, an indication of an outcome of the determining.
23. The method of clause 22, wherein the determining further comprises setting at least one of a threshold of analyte level for the alert condition or an alert enunciation time interval.
24. The method of clause 22 or 23, wherein the determining the advisory message further comprises determining a query for output by the user interface device.
25. The method of clause 24, wherein determining the query comprises determining at least one question about a comfort level of a patient, based on the second time-correlated data indicating a transition from above a high threshold to a low threshold.
26. The method of clause 24 or 25, wherein determining the query comprises determining at least one question about prior patient behavior.
27. The method of any of clauses 22 to 26, further comprising receiving information defining insulin dosing by a patient wearing the sensor control device, wherein the instructions for determining include instructions for correcting an estimate of the patient's plasma insulin state.
28. The method of any of clauses 22 to 27, further comprising causing automatic execution of an insulin dose calculation algorithm in response to receiving a ketone test result.
29. The method of any of clauses 22 to 28, wherein the determining further comprises applying conditional logic defined in a data structure to determine content of the advisory message.
30. The method of any of clauses 22 to 29, wherein the medication dose calculation comprises an insulin dose calculation.
31. The method of clause 30, wherein the determining further comprises basing the insulin dose calculation based on a current ketone level.
32. The method of clause 30 or 31, wherein the insulin dose calculation comprises calculating a mealtime bolus dose.
33. The method of clause 30, 31 or 32, wherein the instructions for determining include instructions for the insulin dose calculation using an adaptive model trained with data from a patient population.
34. The method of any of clauses 30 to 33, wherein the determining further comprises making the insulin dose calculation further based on sensor data indicating a lactic acid measure.
35. The method of any of clauses 22 to 34, wherein the determining the advisory message is further based on whether a patient is dosing with SGLT-2i, and the determining further comprises configuring the advisory message to discontinue use of SGLT-2i if ketones are above a threshold.
36. The method of any of clauses 22 to 35, further comprising wirelessly transmitting the first and the second data to the user interface device.
37. The method of any of clauses 22 to 36, further comprising sending a control signal to an automatic insulin delivery device, based on the determining an insulin dose.
38. An analyte monitoring system, comprising: a sensor control device including an analyte sensor, wherein the analyte sensor includes at least a portion configured to be inserted into a user's body, and wherein the sensor control device is configured to collect first time-correlated data indicative of a glucose level and second time-correlated data indicative of a ketone level; and a reader device,
   the system being configured to perform: determining, based at least in part on the first and second time-correlated data, of at least one of:
   an alert condition for one or both of the first and second time-correlated data, an advisory message for output by the reader device, a correction to an analyte state estimate, a malfunction of the sensor control device, a malfunction of an insulin delivery device, a medication dose calculation, or an analytical report based on the first and second time-correlated data; and
   outputting, by the reader device, an indication of an outcome of the determining.
39. The system of clause 38, further comprising means for performing the determining and outputting.
40. The system of clause 39, wherein the means for performing the determining and outputting comprises processing circuitry and a non-transitory memory.
41. The system of clause 40, wherein the sensor control device comprises processing circuitry and/or a non-transitory memory.
42. The system of clause 39 or 40, wherein the reader device comprises processing circuitry and/or a non-transitory memory.
The claims of the parent application are reproduced below. These clauses define preferable combinations of features. The applicant reserves the right to pursue protection for these combinations of features, and/or any other subject-matter contained in the parent application as filed, either in the present divisional application or in a further application divided from the present divisional application. The claims of the parent application are not the claims of the current application which are contained in a separate section headed "claims".
1. An analyte monitoring system, comprising:
   a sensor control device including an analyte sensor, first processing circuitry, and a first non-transitory memory, wherein the analyte sensor includes at least a portion configured to be inserted into a user's body, and wherein the sensor control device is configured to collect first time-correlated data indicative of a glucose level and second time-correlated data indicative of a ketone level; and
   a reader device comprising second processing circuitry and a second non-transitory memory,
   wherein at least one of the first or the second non-transitory memory includes instructions that, when executed, cause at least one of the first or the second processing circuitry to perform:
      determining, based at least in part on the first and second time-correlated data, at least one of:
         an alert condition for one or both of the first and second time-correlated data, an advisory message for output by the reader device, a correction to an analyte state estimate, a malfunction of the sensor control device, a malfunction of an insulin delivery device, a medication dose calculation, or an analytical report based on the first and second time-correlated data; and
      outputting, by the reader device, an indication of an outcome of the determining.
2. The analyte monitoring system of claim 1, wherein the instructions for determining the alert condition include instructions for setting at least one of a threshold of analyte level for the alert condition or an alert enunciation time interval.
3. The analyte monitoring system of claim 1, wherein the instructions for determining the advisory message include instructions for outputting a query by the reader device.
4. The analyte monitoring system of claim 3, wherein the instructions for outputting the query include instructions for outputting at least one question about a comfort level of a patient, based on the second time-correlated data indicating a transition from above a high threshold to a low threshold.
5. The analyte monitoring system of claim 3, wherein the instructions for outputting the query include instructions for outputting at least one question about prior patient behavior.
6. The analyte monitoring system of claim 1, further comprising an input device operatively coupled to at least one of the reader device or the sensor control device configured for receiving information defining insulin dosing by a patient wearing the sensor control device, wherein the instructions for determining include instructions for correcting an estimate of the patient's plasma insulin state.
7. The analyte monitoring system of claim 6, further comprising an input device operatively coupled to at least one of the reader device or the sensor control device configured for receiving a ketone test result, wherein the instructions further cause automatic execution of an insulin dose calculation algorithm in response to receiving the ketone test result.
8. The analyte monitoring system of claim 1, wherein the instructions for determining include instructions for applying conditional logic defined in a data structure to determine content of the advisory message.
9. The analyte monitoring system of claim 1, wherein the medication dose calculation comprises an insulin dose calculation.
10. The analyte monitoring system of claim 9, wherein the instructions for determining include instructions for the insulin dose calculation based on a current ketone level.
11. The analyte monitoring system of claim 9, wherein the instructions for determining include instructions for the insulin dose calculation comprising a mealtime bolus dose.
12. The analyte monitoring system of claim 9, wherein the instructions for determining include instructions for the insulin dose calculation using an adaptive model trained with data from a patient population.
13. The analyte monitoring system of claim 9, wherein the instructions for determining include instructions for the insulin dose calculation further based on sensor data indicating a lactic acid measure.
14. The analyte monitoring system of claim 1, wherein the instructions for determining the advisory message include instructions for performing the determining further based on whether a patient is dosing with SGLT-2i, and for configuring the advisory message to discontinue use of SGLT-2i if ketones are above a threshold.
15. The analyte monitoring system of claim 1, wherein the instructions are stored on the second non-transitory memory.
16. The analyte monitoring system of claim 1, wherein the instructions are stored on the first non-transitory memory.
17. The analyte monitoring system of claim 1, wherein the sensor control device further includes wireless communications circuitry configured to transmit the first and the second data to the reader device.
18. The analyte monitoring system of claim 16, wherein the wireless communications circuitry is configured to transmit the first and the second data according to a Bluetooth protocol.
19. The analyte monitoring system of claim 1, further comprising a third analyte sensor configured to sense a lactic acid level in the bodily fluid.
20. The analyte monitoring system of claim 1, further comprising a medication delivery device.
21. The analyte monitoring system of claim 19, wherein the medication delivery device comprises an insulin pump.
22. A computer-implemented method for analyte monitoring and control for a patient, the method comprising:
   collecting, by a sensor control device, first time-correlated data indicative of a glucose level and second time-correlated data indicative of a ketone level, wherein the sensor control device includes an analyte sensor at least a portion of which is inserted into a user's body;
   determining, based at least in part on the first and second time-correlated data of at least one of:
      an alert condition for one or both of the first and second time-correlated data, an advisory message for output by the reader device, a correction to an analyte state estimate, a malfunction of the sensor control device, a malfunction of an insulin delivery device, a medication dose calculation, or an analytical report based on the first and second time-correlated data; and
   outputting, by a user interface device, an indication of an outcome of the determining.
23. The method of claim 22, wherein the determining further comprises setting at least one of a threshold of analyte level for the alert condition or an alert enunciation time interval.
24. The method of claim 22, wherein the determining the advisory message further comprises determining a query for output by the user interface device.
25. The method of claim 24, wherein determining the query comprises determining at least one question about a comfort level of a patient, based on the second time-correlated data indicating a transition from above a high threshold to a low threshold.
26. The method of claim 24, wherein determining the query comprises determining at least one question about prior patient behavior.
27. The method of claim 22, further comprising receiving information defining insulin dosing by a patient wearing the sensor control device, wherein the instructions for determining include instructions for correcting an estimate of the patient's plasma insulin state.
28. The method of claim 27, further comprising causing automatic execution of an insulin dose calculation algorithm in response to receiving a ketone test result.
29. The method of claim 22, wherein the determining further comprises applying conditional logic defined in a data structure to determine content of the advisory message.
30. The method of claim 22, wherein the medication dose calculation comprises an insulin dose calculation.
31. The method of claim 30, wherein the determining further comprises basing the insulin dose calculation based on a current ketone level.
32. The method of claim 30, wherein the insulin dose calculation comprises calculating a mealtime bolus dose.
33. The method of claim 30, wherein the instructions for determining include instructions for the insulin dose calculation using an adaptive model trained with data from a patient population.
34. The method of claim 30, wherein the determining further comprises making the insulin dose calculation further based on sensor data indicating a lactic acid measure.
35. The method of claim 22, wherein the determining the advisory message is further based on whether a patient is dosing with SGLT-2i, and the determining further comprises configuring the advisory message to discontinue use of SGLT-2i if ketones are above a threshold.
36. The method of claim 22, further comprising wirelessly transmitting the first and the second data to the user interface device.
37. The method of claim 36, further comprising sending a control signal to an automatic insulin delivery device, based on the determining an insulin dose.

## Claims

1. An analyte monitoring system, comprising:
a sensor control device including an analyte sensor, first processing circuitry, and a first non-transitory memory, wherein the analyte sensor includes at least a portion configured to be inserted into a user's body, and wherein the sensor control device is configured to collect first time-correlated data indicative of a glucose level and second time-correlated data indicative of a ketone level;
a medication delivery device comprising an insulin pump; and
a reader device comprising second processing circuitry and a second non-transitory memory,
wherein at least one of the first or the second non-transitory memory includes instructions that, when executed, cause at least one of the first or the second processing circuitry to perform:
determining, based at least in part on the first and second time-correlated data, an insulin dose calculation comprising a mealtime bolus dose.

2. The analyte monitoring system of claim 1, wherein the instructions for determining include instructions for the insulin dose calculation based on a current ketone level.

3. The analyte monitoring system of claim 1 or 2, wherein the instructions for determining include instructions for the insulin dose calculation based on a current trend in ketone level.

4. The analyte monitoring system of any preceding claim, wherein the instructions for determining include instructions for the insulin dose calculation based on more than one ketone measurement.

5. The analyte monitoring system of any preceding claim, wherein the insulin dose is modified by a function based on ranges of ketone values.

6. The analyte monitoring system of any preceding claim, wherein the insulin dose is calculated using a glycemic response model in which basal insulin, insulin sensitivity and carbohydrate ratio are functions based on the ketone level.

7. The analyte monitoring system of any preceding claim, wherein a model used to calculate the mealtime bolus dose comprises functions based on the current ketone reading, to account for more insulin being required in order to achieve the same effect on glucose when ketones are higher than normal.

8. The analyte monitoring system of any preceding claim, wherein the instructions for an insulin dose calculation comprise using a step-wise linear model to determine insulin sensitivity based on ketone reading.

9. The analyte monitoring system of any preceding claim, wherein the instructions for determining include instructions for the insulin dose calculation based on modeling ketones and higher order derivatives of ketones, along with glucose and high order derivatives of glucose, as state variables.

10. The analyte monitoring system of any preceding claim, configured for receiving information defining insulin dosing for a patient wearing the sensor control device, wherein the instructions for determining include instructions for correcting an estimate of the patient's plasma insulin state.

11. The analyte monitoring system of any preceding claim, wherein the instructions for determining include instructions for the insulin dose calculation using an adaptive model trained with data from a patient population.

12. The analyte monitoring system of any preceding claim, wherein the instructions include instructions for tracking prior insulin dose amounts paired with glucose measurement-derived data and ketone measurement-derived data, and estimating an effect of data prior to the insulin dose on data after the insulin dose.

13. The analyte monitoring system of any preceding claim, wherein the instructions include instructions for using an estimator to update insulin dosing parameters by accounting for both glucose and ketone-derived data up to every insulin dosing decision.

14. The system of any preceding claim, wherein the instructions include instructions for sending a control signal to an automatic insulin delivery device, based on the determining an insulin dose.

15. A computer-implemented method for analyte monitoring and control for a patient, the method comprising:
collecting, by a sensor control device, first time-correlated data indicative of a glucose level and second time-correlated data indicative of a ketone level, wherein the sensor control device includes an analyte sensor at least a portion of which is inserted into a user's body;
determining, based at least in part on the first and second time-correlated data, an insulin dose calculation comprising a mealtime bolus dose.
